# EUROPEAN PATENT APPLICATION

(11) **EP 3 950 099 A1**
(43) Date of publication of application: **09.02.2022**
(21) Application number: 20785104.9
(22) Date of filing: 27.03.2020
(51) Int. Cl.: B01D 61/14, B01D 65/08, B01D 69/00, B01D 69/04, B01D 71/44, C07K 1/34, C07K 1/36, C07K 16/00, B01D 15/00, B01J 20/26, C12P 21/08

(54) **METHOD FOR PURIFYING PROTEIN**

(30) Priority: 29.03.2019 JP 2019065754; 29.03.2019 JP 2019066611
(71) Applicant: Asahi Kasei Medical Co., Ltd., Tokyo 100-0006 (JP)
(72) Inventor: FUTAMURA, Akika, Tokyo 100-0006 (JP); TANIGUCHI, Hiroki, Tokyo 100-0006 (JP); HIDAKA, Junji, Tokyo 100-0006 (JP)
(74) Representative: dompatent von Kreisler Selting Werner - Partnerschaft von Patent- und Rechtsanwälten mbB
(86) International application number: PCT/JP2020/013893
(87) International publication number: WO 2020/203718

(57) **Abstract**

A method for purifying a protein-containing solution comprising a protein of interest, the method comprising the steps of:
(a1) contacting the protein-containing solution with a porous medium comprising polyketone; and
(a2) passing the protein-containing solution contacted with the porous medium comprising polyketone through a virus removal membrane.

## Description

### Technical Field

The present invention relates to a method for recovering a purified protein, and a method for removing a protein aggregate contained in a protein. The present invention also relates to a method for purifying a protein.

### Background Art

Proteins are biopolymers important in industries ranging from food to pharmaceutical industries. Biopharmaceuticals to which such proteins are applied have received attention in recent years in drug development. Among others, particularly, immunoglobulins (antibodies) have received attention and have been increasingly useful for purposes such as medicaments, diagnostic drugs, or materials for separation and purification of the corresponding antigen proteins. The antibodies are physiologically active substances responsible for immune response and are obtained from the blood of immunized animals, cell culture solutions of cells retaining the ability to produce antibodies, or culture solutions of the ascitic fluid of the animals. However, such blood or a culture solution containing the antibody includes proteins other than the antibody, or complicated contaminants derived from a starting material solution used in cell culture. The solution may contain a dimer and higher multimeric aggregates of the antibody.

Examples of the method for removing a protein aggregate include a method of removing the protein aggregate by ultracentrifugation, an approach using an ultrafiltration membrane (see e.g., Patent Literature 1), an approach using a combination of a protein A column, an ion-exchange column, and a size exclusion column (see e.g., Patent Literature 2), and an approach using a hydrophobic gel (see e.g., Non Patent Literature 1). The ultracentrifugation method may be used in the research field, but is not suitable for large-scale production processes. The approach based on an ultrafiltration membrane may have an insufficient degree of purification because of the low fractionation accuracy. Problems of the conventional approach using a column or a gel are cost required for use of expensive columns, possible complicated operations such as gradient elution, degradability ascribable to continuous use, and large burdens due to the dilution of purified proteins on concentration or drying steps after purification. A further problem of the approach using a column or a gel is time-consuming purification of proteins possibly resulting in the denaturation of the proteins.

Aliphatic polyketone (hereinafter, also referred to as "polyketone") is known which is a completely alternate copolymer of carbon monoxide and olefin obtained by polymerizing carbon monoxide and olefin with palladium or nickel as a catalyst. Since the polyketone is highly crystalline, fibers or films formed from the polyketone, for example, have characteristics such as high mechanical properties, high melting points, organic solvent resistance, and chemical resistance. The polyketone is used mainly in, for example, separators for water treatments, gas dust removals, gas separations, and batteries (see e.g., Patent Literatures 3 and 4). Nylon is known as a raw material for media that can remove protein aggregates (see e.g., Patent Literature 5).

Measures to improve virus safety are necessary for biopharmaceuticals. Pharmaceutical manufacturers have studied the introduction of a virus removal/inactivation step into a production process. Among others, a virus removal method by filtration using a virus removal filter is an effective method that can reduce the level of viruses without denaturing useful proteins.

Cases involving parvovirus among other viruses have been reported in the plasma fraction preparation field, including those of infection by human parvovirus B19 and those of contamination of CHO (Chinese hamster ovary) cells by mouse parvovirus. For example, cases where microscopic bacteria such as *Leptospira* species remain in biopharmaceuticals have also been reported.

The parvovirus, which is a small virus, has no envelope and is therefore physicochemically stable and resistant to heating, low pH, or chemical treatment, which is an inactivation step generally performed in medicament production processes. Hence, there are growing needs for virus removal and bacterium removal with virus removal membranes as virus removal methods based on the mechanism of action different from that of the inactivation method.

Membranes made of natural materials such as cellulose or virus removal membranes made of synthetic polymer materials such as polyvinylidene fluoride (PVDF) or polyethersulfone (PES) are known as filtration membranes for virus removals (see e.g., Patent Literatures 6 and 7 or Non Patent Literatures 2 to 5).

### Citation List

### Patent Literature

Patent Literature 1: Japanese Patent Laid-Open No. 56-59716
Patent Literature 2: Japanese Patent No. 2638680
Patent Literature 3: Japanese Patent Laid-Open No. 2015-203048
Patent Literature 4: International Publication No. WO 2013/035747
Patent Literature 5: Japanese Patent Laid-Open No. 2005-145852
Patent Literature 6: U.S. Patent Application Publication No. 2016/0176921
Patent Literature 7: International Publication No. WO 2016/031834
Patent Literature 8: Japanese Patent Laid-Open No. 2014-151272
Patent Literature 9: International Publication No. WO 2003/055934
Patent Literature 10: International Publication No. WO 2016/031834

### Non Patent Literature

Non Patent Literature 1: HLC MAILGRAM, Tosoh Corp., August 25, 2003, Vol. 97, No. 3, p. 10
Non Patent Literature 2: Manabe. S, Removal of virus through novel membrane filtration method., Dev. Biol. Stand., (1996), 88: 81-90
Non Patent Literature 3: Brandwein H et al., Membrane filtration for virus removal., Dev Biol (Basel)., (2000), 102: 157-63
Non Patent Literature 4: Aranha-Creado et al., Clearance of murine leukaemia virus from monoclonal antibody solution by a hydrophilic PVDF microporous membrane filter., Biologicals. (1998) Jun; 26 (2): 167-72
Non Patent Literature 5: L. Moce-Llivina et al., Comparison of polyvinylidene fluoride and polyether sulfone membranes in filtering viral suspensions, Journal of Virological Methods, (2003) April, Vol. 109, Issue 1, Pages 99-101

### Summary of Invention

### Technical Problem

An object of the present invention is to provide a method for recovering a purified protein. Another object of the present invention is to provide a method for removing a protein aggregate contained in a protein preparation.

The present inventors have also found the problem of difficulty in controlling a purification process due to the generation of a fraction of a protein aggregate with a concentration higher than an initial concentration in the course of passing a protein solution through a medium comprising nylon. Accordingly, an alternative object of the present invention is to provide a method for purifying a protein by an easy-to-control purification process. A further alternative object of the present invention is to provide a novel medium suitable for each method.

In formulation processes of biopharmaceuticals, virus removal membranes are often used in the final steps of the formulation processes. In such a step using a virus removal membrane, a purification method that causes less pressure loss in a prefilter step and is capable of efficiently removing a protein aggregate or the like is preferred because a throughput per unit time would be decreased if pressure loss occurs in a step prior thereto (prefilter step, etc.). As virus removal membranes are often used in the final steps of formulation processes, it is preferred that the filtration of protein preparations through the virus removal membranes be able to treat larger amounts of proteins in a short time and to remove viruses with sufficiently high virus removal performance. However, the protein preparations often contain biopolymers serving as substances causative of the fouling of virus removal membranes and may cause blocking of the virus removal membranes. Thus, an another object of the present invention is to provide a method for purifying a protein by reducing the fouling (blocking) of a virus removal membrane during protein purification with the virus removal membrane.

### Solution to Problem

The present inventors have conducted diligent studies to attain the objects and consequently found a method for purifying or recovering a protein with a medium comprising polyketone. The present inventors have also found that the medium comprising polyketone is capable of removing a protein aggregate. The present inventors have further conducted diligent studies and consequently found that use of the medium comprising polyketone facilitates controlling a purification process without causing a phenomenon in which a fraction of a protein aggregate with a concentration higher than an initial concentration, which is found in the case of using a medium comprising nylon.

In addition, the present inventors have found that it is possible to reduce the fouling of the virus removal membrane and to purify the protein efficiently by contacting a protein solution of interest with a specific porous medium before filtration of the protein solution through a virus removal membrane.

Accrodingly, the present invention includes the following.
[A1] A method for purifying a protein-containing solution comprising a protein of interest, the method comprising the steps of:
   (α1) contacting the protein-containing solution with a porous medium comprising polyketone; and
   (α2) passing the protein-containing solution contacted with the porous medium comprising polyketone through a virus removal membrane.
[A1-2] The method according to [A1], wherein the polyketone is polyketone comprising 70 mol% or more of a 1-oxotrimethylene repeat unit.
[A2] A method for purifying a protein-containing solution comprising a protein of interest, the method comprising the steps of:
   (β1) contacting the protein-containing solution with a porous medium having a specific surface area of 4.0 m²/mL or larger and a porosity of 50% or more; and
   (β2) passing the protein-containing solution contacted with the porous medium through a virus removal membrane.
[A3] The method according to [A1], [A1-2] or [A2], wherein the porous medium is in a membrane form.
[A4] The method according to any of [A1] to [A3], wherein the porous medium and the virus removal membrane are connected directly.
   Cited item numbers are indicated by a range, as in [A1] to [A3] described above, and an item having a branch number such as [A1-2] may be included within the range. This means that the item having a branch number such as [A1-2] is also cited. The same holds true for the description below.
[A5] The method according to [A4], wherein a pressure applied to the porous body used in the step (α1) or (β1) is 1/40 to 1/2 of a pressure applied to the virus removal membrane used in the step (α2) or (β2).
[A6] The method according to any of [A1] to [A5], wherein filtration is performed at a constant pressure.
[A7] The method according to any of [A1] to [A6], wherein a virus removal rate from the protein-containing solution is at least 99.9%.
[A8] The method according to any of [A1] to [A7], wherein the protein of interest is a polyclonal antibody or a monoclonal antibody.
[A9] The method according to any of [A1] to [A8], wherein a protein concentration of the protein-containing solution is 100 mg/mL or lower.
[A10] The method according to any of [A1] to [A9], wherein the virus to be removed with the virus removal membrane is parvovirus.
[A11] The method according to any one of [A1] and [A3] to [A10], wherein a specific surface area (polyketone surface area (m²) / polyketone volume (mL)) of the polyketone is 12 m²/mL or larger.
[A12] A method for producing a protein-containing solution substantially free from a virus by a method according to any of [A1] to [A11].
[A13] A prefilter for a virus removal membrane, comprising a porous medium comprising polyketone.
[A13-2] The prefilter for a virus removal membrane according to [A13], wherein the polyketone is polyketone comprising 70 mol% or more of a 1-oxotrimethylene repeat unit.
[A14] Use of polyketone for a prefilter for a virus removal membrane.
[A15] Use of polyketone for the production of a prefilter for a virus removal membrane.
[A16] The use according to [A14] or [A15], wherein the polyketone is polyketone comprising 70 mol% or more of a 1-oxotrimethylene repeat unit.
[B1] A method for recovering a purified protein from a protein-containing solution, the method comprising the steps of:
   (a1) contacting the protein-containing solution with a medium comprising polyketone; and
   (a2) recovering the purified protein from the protein-containing solution contacted with the medium comprising polyketone.
[B1-2] The method according to [B1], wherein the polyketone is polyketone comprising 70 mol% or more of a 1-oxotrimethylene repeat unit.
[B2] A method for selectively removing a protein aggregate contained in a protein-containing solution from the protein-containing solution, the method comprising the step of:
   (b1) contacting the protein-containing solution with a medium comprising polyketone.
[B3] The method according to [B1], [B1-2] or [B2], wherein the protein is an antibody.
[B4] The method according to [B3], wherein the antibody is an antibody derived from a plasma product or a cell culture solution.
[B5] The method according to [B3], wherein the antibody is a monoclonal antibody.
[B6] The method according to any one of [B2] to [B5], wherein the protein aggregate is a dimer, a trimer, and/or a multimer of the protein.
[B7] The method according to any one of [B1] to [B6], wherein the step (a1) or (b1) is the step of passing the protein-containing solution through the medium comprising polyketone.
[B8] The method according to any one of [B1] to [B7], wherein pH of the protein-containing solution is 4 or higher and 10 or lower.
[B9] The method according to any one of [B1] to [B8], wherein an electrical conductivity of the protein-containing solution is 0 mS/cm or more and 100 mS/cm or less.
[B10] The method according to any one of [B1] to [B9], wherein a flow rate at which the protein-containing solution is passed through the medium comprising polyketone is 0.1 mL/min or more per mL of a membrane volume of the medium comprising polyketone.
[B11] The method according to any one of [B1] to [B10], wherein a surface area of the medium comprising polyketone is at least 0.0001 m² or larger per 1.0 g of the protein to be purified.
[B12] The method according to any one of [B1] to [B11], wherein the medium comprising polyketone is a porous molded body.
[B13] The method according to [B12], wherein the porous molded body is a microporous membrane, a non-woven fabric, a woven fabric, a monolith, a gel, or a particle bed.
[B14] The method according to [B12] or [B13], wherein the porous molded body has a three-dimensionally uniform membrane pore size.
[B15] The method according to any one of [B12] to [B14], wherein a pore size of the porous molded body is 50 nm or larger and 1000 nm or smaller.
[B16] A medium for selectively removing a protein aggregate from a protein-containing solution, the medium comprising polyketone.
[B16-2] The medium according to [B16], wherein the polyketone is polyketone comprising 70 mol% or more of a 1-oxotrimethylene repeat unit.
[B17] The medium according to [B16] or [B16-2], wherein the protein is an antibody.
[B18] The medium according to [B17], wherein the antibody is an antibody derived from a plasma product or a cell culture solution.
[B19] The medium according to [B17], wherein the antibody is a monoclonal antibody.
[B20] The medium according to any one of [B16] to [B19], wherein the protein aggregate is a dimer, a trimer, and/or a multimer of the protein.
[B21] The medium according to any one of [B16] to [B20], wherein the medium comprising polyketone is a porous molded body.
[B22] The medium according to [B21], wherein the porous molded body is a microporous membrane, a non-woven fabric, a woven fabric, a monolith, a gel, or a particle bed.
[B23] The medium according to [B21] or [B22], wherein the porous molded body has a three-dimensionally uniform membrane pore size.
[B24] The medium according to any one of [B21] to [B23], wherein a pore size of the porous molded body is 50 nm or larger and 1000 nm or smaller.
[B25] Use of polyketone for selectively removing a protein aggregate from a protein-containing solution.
[B25-2] The use according to [B25], wherein the polyketone is polyketone comprising 70 mol% or more of a 1-oxotrimethylene repeat unit.

### Advantageous Effects of Invention

According to the present invention, a purified protein of interest can be efficiently recovered. Furthermore, according to the present invention, a protein aggregate contained in a preparation of a protein of interest can be efficiently removed. Moreover, according to the present invention, protein purification (aggregate removal) of interest can be relatively easily controlled. In addition, the present invention can provide a medium suitable for the protein purification. The present invention can further provide a method for purifying a protein by reducing the fouling (blocking) of a virus removal membrane during protein purification with the virus removal membrane.

### Brief Description of Drawings

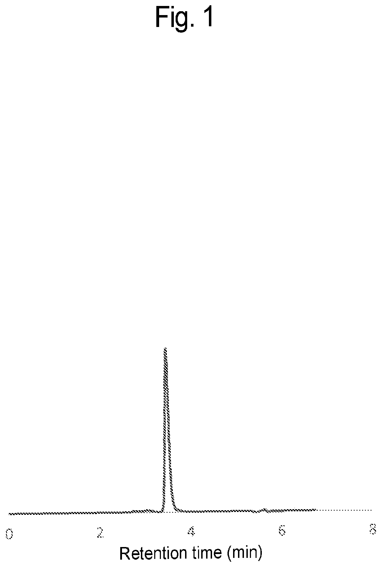

[Figure 1] Figure 1 is a chromatography chart according to Example B1.

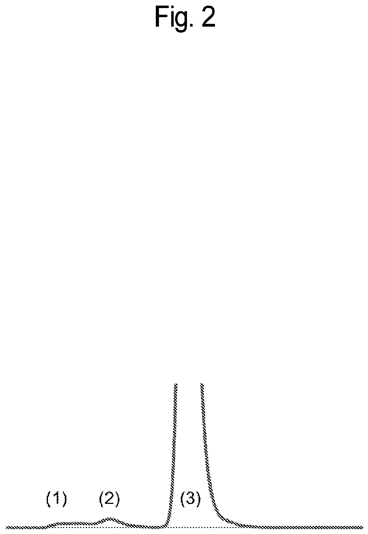

[Figure 2] Figure 2 is an enlarged view of a portion of the chromatography chart shown in Figure 1.

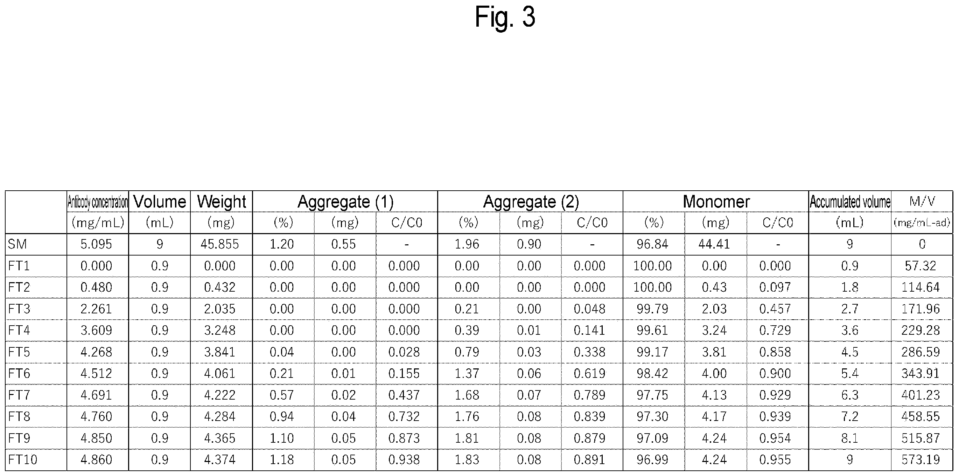

[Figure 3] Figure 3 is a table showing the content weights of aggregates and a monomer contained in each fraction according to Example B1.

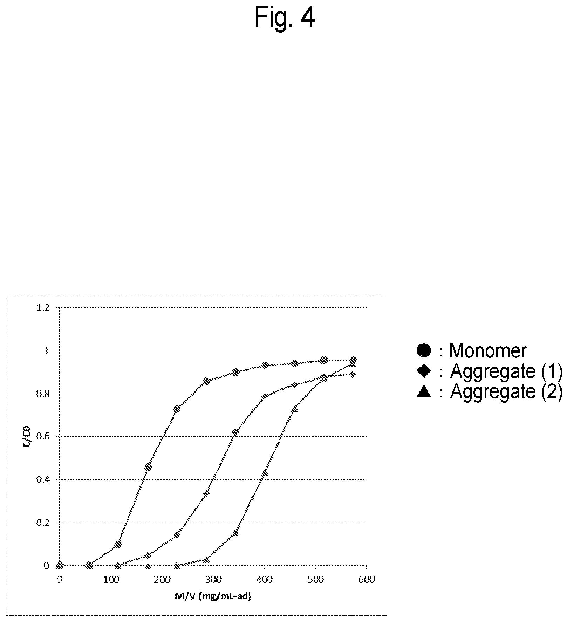

[Figure 4] Figure 4 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B1.

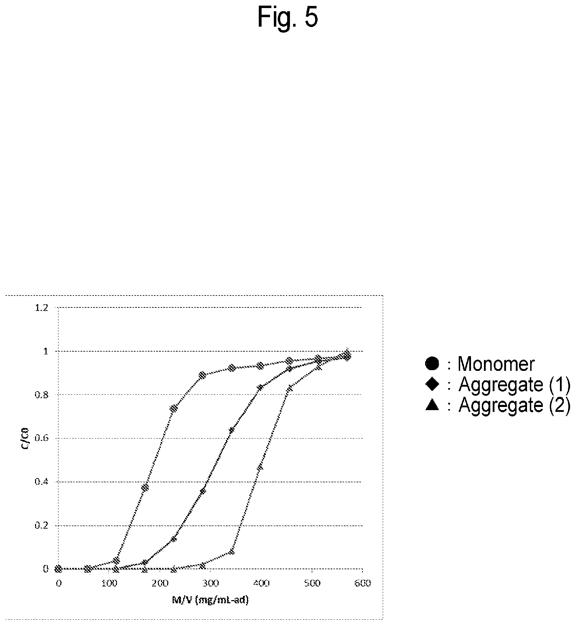

[Figure 5] Figure 5 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B2.

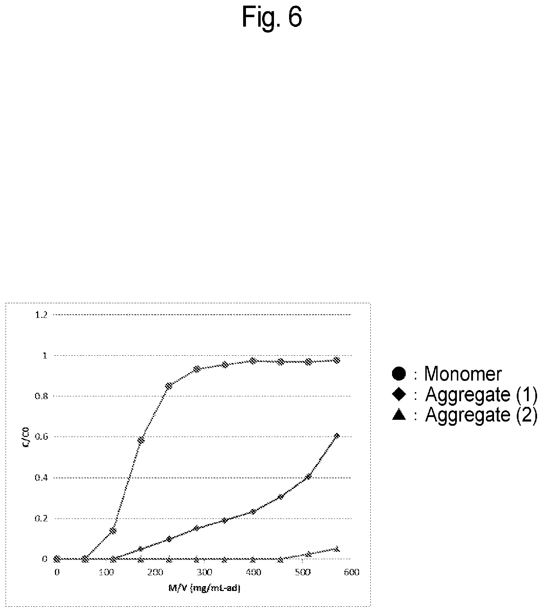

[Figure 6] Figure 6 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B3.

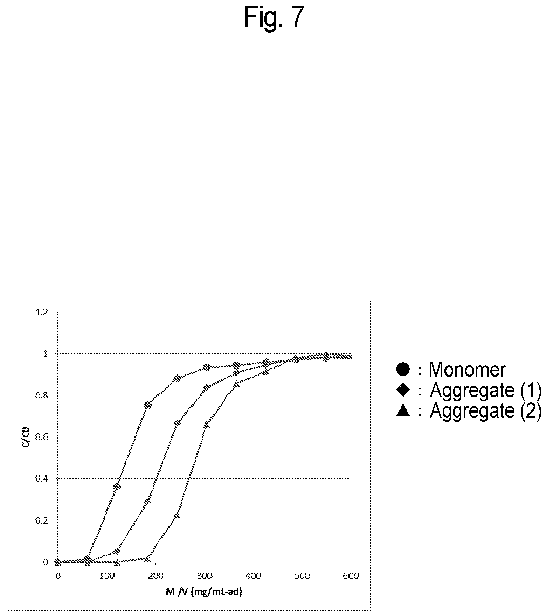

[Figure 7] Figure 7 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B4.

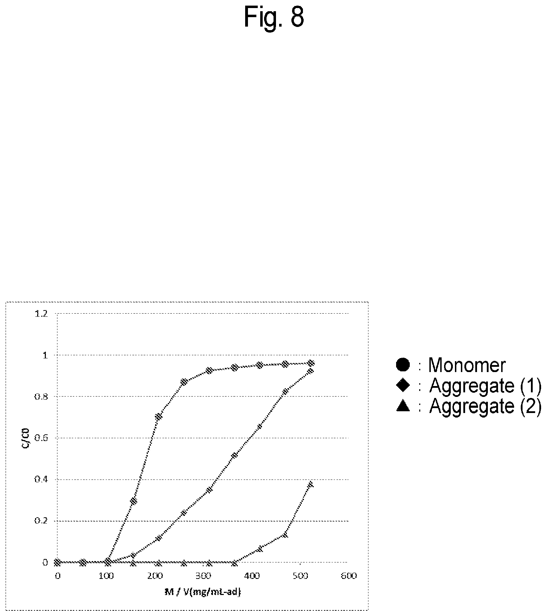

[Figure 8] Figure 8 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B5.

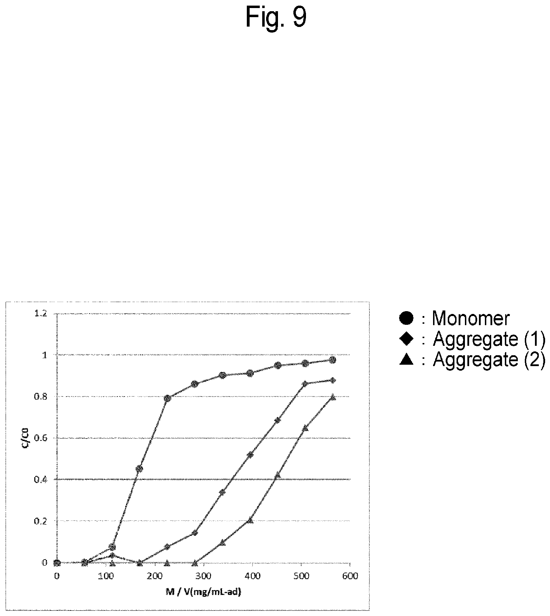

[Figure 9] Figure 9 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B6.

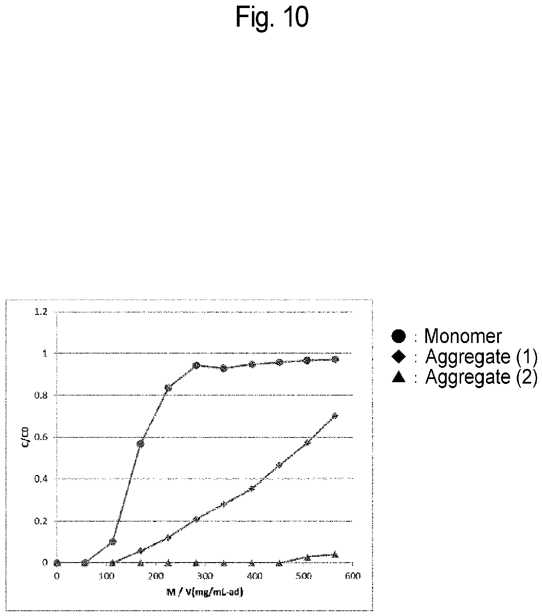

[Figure 10] Figure 10 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B7.

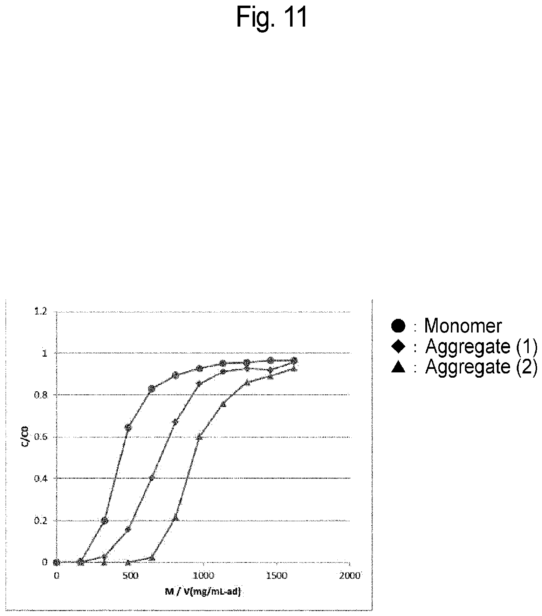

[Figure 11] Figure 11 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B8.

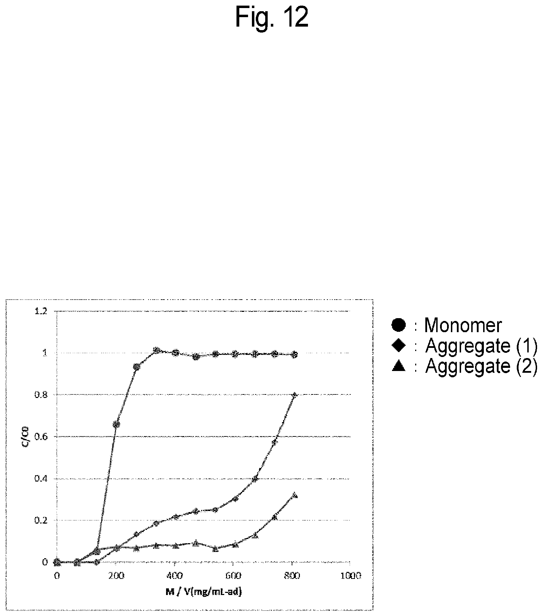

[Figure 12] Figure 12 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B9.

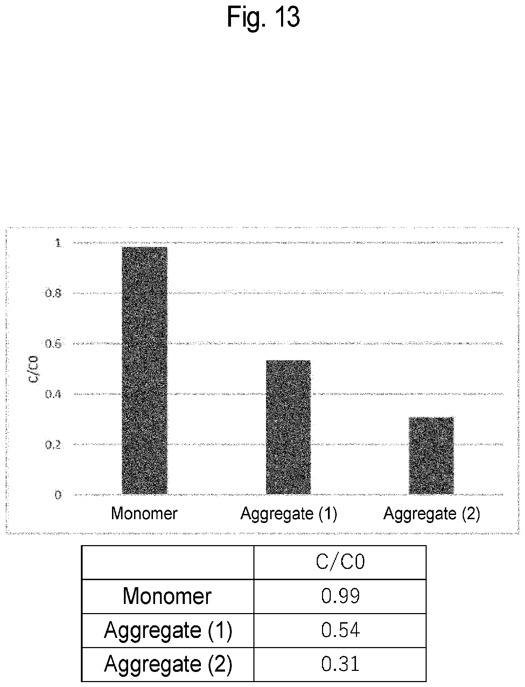

[Figure 13] Figure 13 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Example B10.

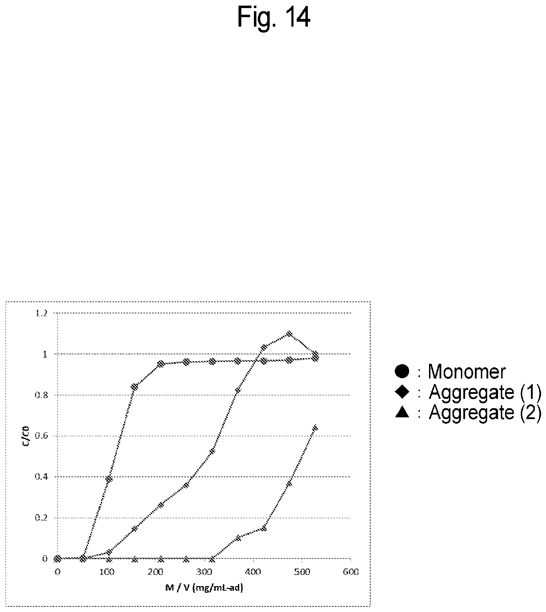

[Figure 14] Figure 14 is a graph showing the relationship between a value obtained by dividing the accumulated weight of antibodies passed through an evaluation subject by the membrane volume of the evaluation subject, and the ratio of the concentration of a monomer or an aggregate in a fraction to the concentration of the monomer or the aggregate before passage through the evaluation subject, according to Comparative Example B1.

### Description of Embodiments

Hereinafter, the embodiments of the present invention (hereinafter, also referred to as the "embodiments" in the present specification) will be specifically described. The embodiments given below illustrate methods, etc. for concretizing the technical brief of this invention and are not limited to these examples.

### 1. Method for recovering purified protein

The method for recovering a purified protein from a protein-containing solution according to an embodiment comprises the steps of: (a1) contacting the protein-containing solution with a medium comprising polyketone; and (a2) recovering the purified protein from the protein-containing solution contacted with the medium comprising polyketone.

In one embodiment, the polyketone is a copolymer of carbon monoxide and one or more types of olefins. Examples of the polyketone include a polymer represented by the following formula (1):

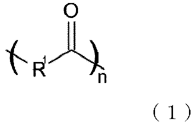

Examples of the polyketone include a polymer in which a repeat unit containing a R¹ group optionally substituted with a carbonyl group is alternately arranged. In the formula (1), examples of R¹ include an alkylene group having carbon number of 2 or more and 20 or less. The substituent for the R¹ group can include one or more substituents selected from the group consisting of a hydrogen atom, a halogen atom, a hydroxy group, an ether group, a primary amino group, a secondary amino group, a tertiary amino group, a quaternary ammonium group, a sulfonate group, a sulfonic acid ester group, a carboxylate group, a carboxylic acid ester group, a phosphate group, a phosphoric acid ester group, a thiol group, a sulfide group, an alkoxysilyl group, and a silanol group. The polymer may contain a unit other than the repeat unit. An example of the upper limit value of the content of the unit other than the repeat unit is less than 10% by weight in the polyketone. In an alternative aspect, an example thereof is less than 9% by weight. In an alternative aspect, an example thereof is less than 8% by weight. In an alternative aspect, an example thereof is less than 7% by weight. In an alternative aspect, an example thereof is less than 6% by weight. In an alternative aspect, an example thereof is less than 5% by weight. In an alternative aspect, an example thereof is less than 4% by weight. In an alternative aspect, an example thereof is less than 3% by weight. In an alternative aspect, an example thereof is less than 2% by weight. In an alternative aspect, an example thereof is less than 1% by weight. In a further alternative aspect, an example thereof is less than 0.5% by weight. An example of the lower limit value of the content of the unit other than the repeat unit is 0% by weight or more. In an alternative aspect, an example thereof is 0.1% by weight or more. In an alternative aspect, an example thereof is 0.2% by weight or more. In an alternative aspect, an example thereof is 0.3% by weight or more. In an alternative aspect, an example thereof is 0.4% by weight or more. In a further alternative aspect, an example thereof is 0.5% by weight or more. In the polymer, carbonyl groups or alkylene groups may be partially bonded to each other. An alternating copolymer in which a carbonyl group is bonded next to R¹ and R¹ is bonded next to the carbonyl group is referred to as a completely alternating copolymer. An example of the lower limit value of the content of the completely alternating copolymer moiety in the polyketone is 95% by weight or more. In an alternative aspect, an example thereof is 96% by weight or more. In a further alternative aspect, an example thereof is 97% by weight or more. In an alternative aspect, an example thereof is 98% by weight or more. In a further alternative aspect, an example thereof is 99% by weight or more. An example of the upper limit value of the content of the completely alternating copolymer moiety in the polyketone is 100% by weight or less. In an alternative aspect, an example thereof is 99% by weight or less. In a further alternative aspect, an example thereof is 98% by weight or less. In a further alternative aspect, an example thereof is 97% by weight or less. An aspect in which the content of the completely alternating copolymer in the polyketone is 95% by weight or more is preferred from the viewpoint of improving heat resistance or light resistance.

An example of the carbon number in the R¹ group in the formula (1) is 2 or more and 8 or less. In an alternative aspect, an example thereof is 2 or more and 3 or less. In a further alternative aspect, an example thereof is 2. The repeat unit constituting the polyketone is particularly preferably rich in a 1-oxotrimethylene repeat unit represented by the formula (2) given below. The R¹ group in the formula (1) may or may not contain an aromatic ring.

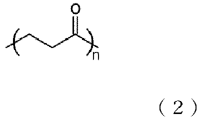

The proportion of the 1-oxotrimethylene repeat unit in the repeat unit constituting the polyketone is preferably 70 mol% or more, more preferably 90 mol% or more, further preferably 95 mol% or more, from the viewpoint of securing the strength of the medium comprising polyketone. The ratio of the 1-oxotrimethylene repeat unit may be 100 mol%. In this context, the term "100 mol%" means that no repeat unit other than 1-oxotrimethylene is observed in the polymer except for its terminal group in an analysis method known in the art such as elemental analysis, NMR (nuclear magnetic resonance), or gas chromatography. Typically, the structure of the repeat unit constituting the polyketone and the amount of each structure can be confirmed by NMR.

In one embodiment, the shape of the medium comprising polyketone (hereinafter, also referred to as a "polyketone medium") is not particularly limited as long as the shape permits protein purification. Examples thereof include shapes such as beads, gels, sponges, and powders. In an alternative aspect, an example thereof is a fiber or a membrane (flat membrane or hollow fiber membrane). In an alternative aspect, an example of the shape of the polyketone medium is a flat membrane or a hollow fiber membrane. In a further alternative aspect, an example thereof is a flat membrane. In a further alternative aspect, an example thereof is a hollow fiber membrane. The polyketone medium may be a porous molded body. The porous molded body may be a microporous membrane, a non-woven fabric, a woven fabric, a monolith, a gel, or a particle bed.

In one embodiment, in the case where the polyketone medium is a membrane, the membrane volume is not particularly limited as long as the membrane volume permits protein purification. An example of the lower limit value of the membrane volume is 0.10 mL or larger per 1.0 g of the protein to be purified. In an alternative aspect, an example thereof is 0.50 mL or larger. In an alternative aspect, an example thereof is 1.0 mL or larger. In a further alternative aspect, an example thereof is 1.5 mL or larger. An example of the upper limit value of the membrane volume is 100000 mL or smaller per 1.0 g of the protein to be purified. In an alternative aspect, an example thereof is 10000 mL or smaller. In an alternative aspect, an example thereof is 1000 mL or smaller. In an alternative aspect, an example thereof is 100 mL or smaller. In a further alternative aspect, an example thereof is 10 mL or smaller.

In one embodiment, the surface area of the polyketone medium is not particularly limited as long as the surface area permits protein purification. An example of the lower limit value of the surface area is 0.0001 m² or larger per 1.0 g of the protein to be purified. In an alternative aspect, an example thereof is 0.001 m² or larger. In an alternative aspect, an example thereof is 0.01 m² or larger. In an alternative aspect, an example thereof is 0.1 m² or larger. In an alternative aspect, an example thereof is 1.0 m² or larger. In a further alternative aspect, an example thereof is 5.0 m² or larger. An example of the upper limit value of the surface area is 1000000 m² or smaller per 1.0 g of the protein to be purified. In an alternative aspect, an example thereof is 100000 m² or smaller. In an alternative aspect, an example thereof is 10000 m² or smaller. In an alternative aspect, an example thereof is 1000 m² or smaller. In an alternative aspect, an example thereof is 100 m² or smaller. In a further alternative aspect, an example thereof is 30 m² or smaller. The surface area is calculated from a specific surface area. The specific surface area means the surface area of the polyketone per unit volume of the polyketone medium. The surface area is calculated from the specific surface area and is the product of the specific surface area and the volume of the polyketone medium.

In the case where the polyketone medium is a porous molded body, the pore size of the polyketone medium is not particularly limited as long as the pore size permits protein purification. An example of the upper limit value of the pore size is 1000 nm or smaller. In an alternative aspect, an example thereof is 900 nm or smaller. In an alternative aspect, an example thereof is 800 nm or smaller. In an alternative aspect, an example thereof is 700 nm or smaller. In an alternative aspect, an example thereof is 600 nm or smaller. In an alternative aspect, an example thereof is 500 nm or smaller. In a further alternative aspect, an example thereof is 400 nm or smaller. An example of the lower limit value of the pore size is 50 nm or larger. In an alternative aspect, an example thereof is 60 nm or larger. In an alternative aspect, an example thereof is 70 nm or larger. In an alternative aspect, an example thereof is 80 nm or larger. In an alternative aspect, an example thereof is 90 nm or larger. In a further alternative aspect, an example thereof is 100 nm or larger.

The pore size distribution of the polyketone medium may be three-dimensionally uniform or non-uniform. The three-dimensionally uniform membrane pore size can be confirmed from a three-dimensional scanning electron microscope (SEM) image of the membrane. In the SEM measurement, for example, FIB-SEM FEI Helios NanoLab 600 (manufactured by FEI Company) can be used. Examples of the measurement approach include a method described in the paragraph [0032] of Patent Literature 8.

The method for producing the polyketone serving as a starting material for porous membranes is not particularly limited. The polyketone can be produced by a method known in the art as described in, for example, Patent Literature 9. In one embodiment, the polyketone is obtained by suspension-polymerization of an ethylenic unsaturated compound and carbon monoxide in the presence of a catalyst in a liquid medium. The liquid medium may be any liquid that may be used in suspension polymerization for the polyketone. A water-soluble organic solvent is preferred from the viewpoint of polymerization activity, fluidity and handleability. Examples of the liquid medium can include alcohols such as methanol, ethanol, propanol, isopropanol, butanol, hexafluoroisopropanol, and ethylene glycol, phenols such as m-cresol, amines such as aniline, ketones such as acetone and methyl ethyl ketone, ethers such as diethyl ether, ethylene glycol, polyethylene glycol, tetrahydrofuran, and diglyme, nitriles such as acetonitrile, acetic acid, and esters such as methyl acetate. These solvents may each be used singly or may be used as a mixed solvent of two or more thereof.

In one embodiment, the protein to be purified is not particularly limited as long as the protein allows a protein monomer and a protein aggregate to be separated with the polyketone medium. Examples of the protein to be purified include albumin, globulin, and fibrinogen. In an alternative aspect, an example of the protein to be purified is an antibody.

In one embodiment, examples of the antibody, one example of physiologically active substances, include glycoprotein molecules (also referred to as gamma globulins or immunoglobulins) that are produced by B lymphocytes as the infection protective mechanism of vertebrates, as generally defined in biochemistry. For example, the antibody to be purified in an embodiment is used as a medicament for humans and has substantially the same structure as that of the antibody in the body of human, the target for administration.

The antibody may be a human antibody or may be an antibody protein derived from a non-human mammal such as bovine or a mouse. Alternatively, the antibody may be a chimeric antibody protein with human IgG or a humanized antibody. The chimeric antibody with human IgG is an antibody having variable regions derived from a non-human organism such as a mouse and constant regions replaced with those of a human-derived immunoglobulin. The humanized antibody is an antibody having complementarity-determining regions (CDRs) derived from a non-human organism and framework regions (FRs) derived from a human in variable regions. The humanization further reduces immunogenicity beyond the chimeric antibody.

The class (isotype) or subclass of the antibody is not particularly limited. For example, the antibody is classified into five classes, IgG, IgA, IgM, IgD, and IgE, depending on difference in the structures of constant regions. However, the antibody to be purified by a filtration method according to an embodiment may be of any of these five classes. The human antibody has four subclasses, IgG1 to IgG4, for IgG, and two subclasses, IgA1 and IgA2, for IgA. However, the antibody to be purified by a filtration method according to an embodiment may be of any of the subclasses. An antibody-related protein such as an Fc-fusion protein in which a protein is attached to an Fc region can be included in the antibody to be purified by a filtration method according to an embodiment.

The antibody can also be classified depending on an origin. However, the antibody to be purified by a filtration method according to an embodiment may be any of a natural human antibody, a recombinant human antibody produced by a genetic recombination technology, a monoclonal antibody, and a polyclonal antibody. Among these antibodies, a monoclonal antibody is suitable as the antibody to be purified by a filtration method according to an embodiment from the viewpoint of demand and importance as antibody drugs, though the antibody is not limited thereto.

Examples of the antibody include monoclonal antibodies and polyclonal antibodies including any of IgM, IgD, IgG, IgA, and IgE. The antibody may be derived from a plasma product or may be derived from a cell culture solution, for example. In the case of obtaining the antibody by cell culture, animal cells or a microbe can be used as cells. The animal cells are not particularly limited by their type. Examples thereof include CHO cells, Sp2/0 cells, NS0 cells, Vero cells, and PER.C6 cells. The microbe is not particularly limited by its type. Examples thereof include *E. coli* and yeasts.

In one embodiment, the proteins before filtration through the polyketone medium may include a biopolymer, in addition to a monomer of the protein of interest. The biopolymer is not particularly limited as long as the biopolymer is a substance causative of the fouling of a virus removal filter. An example thereof is an aggregate of the protein of interest. In an alternative aspect, an example thereof is an aggregate of the protein of interest and a protein other than the protein of interest. In one embodiment, examples of the protein aggregate include aggregates formed by aggregating a plurality of monomers of the protein. The biopolymer is also referred to as a biopolymer assembly.

In one embodiment, the molecular weight of the included protein monomer is not particularly limited as long as the polyketone medium is capable of separating proteins into a protein monomer and a protein aggregate. An example of the lower limit value of the molecular weight of the included protein monomer is 0.1 kDa or higher. In an alternative aspect, an example thereof is 1 kDa or higher. In an alternative aspect, an example thereof is 10 kDa or higher. In an alternative aspect, an example thereof is 50 kDa or higher. In a further alternative aspect, an example thereof is 100 kDa or higher. An example of the upper limit value of the molecular weight is 10000 kDa or lower. In an alternative aspect, an example thereof is 5000 kDa or lower. In an alternative aspect, an example thereof is 1000 kDa or lower. In an alternative aspect, an example thereof is 500 kDa or lower. In a further alternative aspect, an example thereof is 200 kDa or lower.

In one embodiment, the protein-containing solution is not particularly limited as long as proteins are dissolved in a solution. Examples of the type of a buffer solution that can be used as the solution include, but are not particularly limited to, buffer solutions containing tris salt, acetate, Tween, sorbitol, maltose, glycine, arginine, lysine, histidine, sulfonate, phosphate, citric acid, or sodium chloride dissolved therein.

In one embodiment, the concentration of the protein solution is not particularly limited as long as proteins are dissolved in a solution. An example of the lower limit value of the protein solution concentration is 0.01 mg/mL or higher. In an alternative aspect, an example thereof is 0.05 mg/mL or higher. In an alternative aspect, an example thereof is 0.1 mg/mL or higher. In an alternative aspect, an example thereof is 0.5 mg/mL or higher. In an alternative aspect, an example thereof is 1.0 mg/mL or higher. In a further alternative aspect, an example thereof is 5.0 mg/mL or higher. An example of the upper limit value of the protein solution concentration is 100 mg/mL or lower. In an alternative aspect, an example thereof is 90 mg/mL or lower. In an alternative aspect, an example thereof is 80 mg/mL or lower. In an alternative aspect, an example thereof is 70 mg/mL or lower. In an alternative aspect, an example thereof is 60 mg/mL or lower. In an alternative aspect, an example thereof is 50 mg/mL or lower. In an alternative aspect, an example thereof is 40 mg/mL or lower. In an alternative aspect, an example thereof is 30 mg/mL or lower. In an alternative aspect, an example thereof is 25 mg/mL or lower. In a further alternative aspect, an example thereof is 20 mg/mL or lower.

In one embodiment, the concentration of the buffer solution is not particularly limited as long as the material to be dissolved mentioned above is dissolved therein. An example of the lower limit value of the concentration of the buffer solution is 0 mmol/L or higher. In an alternative aspect, an example thereof is 0.5 mmol/L or higher. In an alternative aspect, an example thereof is 1.0 mmol/L or higher. In an alternative aspect, an example thereof is 5 mmol/L or higher. In an alternative aspect, an example thereof is 10 mmol/L or higher. In an alternative aspect, an example thereof is 15 mmol/L or higher. In a further alternative aspect, an example thereof is 25 mmol/L or higher.

In one embodiment, the pH of the protein solution or the buffer solution is not particularly limited as long as the pH allows a protein monomer and a protein aggregate to be separated with the polyketone medium. An example of the lower limit value of the pH is 4.0 or higher. In an alternative aspect, an example thereof is 4.5 or higher. In a further alternative aspect, an example thereof is 5.0 or higher. In a further alternative aspect, an example thereof is 5.5 or higher. In a further alternative aspect, an example thereof is 6.0 or higher. An example of the upper limit value of the pH is 10.0 or lower. In an alternative aspect, an example thereof is 9.0 or lower. In a further alternative aspect, an example thereof is 8.0 or lower. In a further alternative aspect, an example thereof is 8.5 or lower. In a further alternative aspect, an example thereof is 8.0 or lower. In a further alternative aspect, an example thereof is 7.5 or lower. In a further alternative aspect, an example thereof is 7.0 or lower. Examples of the method for measuring the pH include, but are not particularly limited to, methods using hydrogen electrodes, quinhydrone electrodes, antimony electrodes, or glass electrodes. Among them, a method using a glass electrode is preferred.

The electrical conductivity of the protein solution or the buffer solution is not particularly limited as long as the electrical conductivity allows a protein monomer and a protein aggregate to be separated with the polyketone medium. An example of the lower limit value of the electrical conductivity is 0 mS/cm or more. In an alternative aspect, an example thereof is 1 mS/cm or more. In an alternative aspect, an example thereof is 2 mS/cm or more. In an alternative aspect, an example thereof is 3 mS/cm or more. In an alternative aspect, an example thereof is 4 mS/cm or more. In a further alternative aspect, an example thereof is 5 mS/cm or more. An example of the upper limit value of the electrical conductivity is 100 mS/cm or less. In an alternative aspect, an example thereof is 90 mS/cm or less. In a further alternative aspect, an example thereof is 80 mS/cm or less. In a further alternative aspect, an example thereof is 70 mS/cm or less. In a further alternative aspect, an example thereof is 60 mS/cm or less. In a further alternative aspect, an example thereof is 50 mS/cm or less. In a further alternative aspect, an example thereof is 40 mS/cm or less. The method for measuring the electrical conductivity is not particularly limited. For example, a method stipulated in JIS K0130 "General rules for electrical conductivity measuring method " is available. Examples of such a method include an alternating current two-electrode scheme, an alternating current four-electrode scheme, and an electromagnetic induction scheme. Among them, an alternating current two-electrode scheme is preferred.

In one embodiment, the purified protein is not particularly limited as long as the purified protein is a protein with the content of an aggregate (dimer or multimer) of the protein of interest reduced from the content of the aggregate of the protein of interest in proteins in the protein-containing solution before purification, i.e., before contact with the polyketone.

In one embodiment, examples of the protein aggregate include a dimer formed by association of two monomers of the protein of interest, a trimer formed by association of three monomers of the protein of interest, and a multimer formed by association of four or more monomers of the protein of interest, and mixtures thereof. The multimer may include a dimer and a trimer. The protein aggregate may contain a protein other than the protein of interest. The protein aggregate may be an irreversible associate or may be a reversible associate.

In one embodiment, the recovery of the purified protein is not particularly limited as long as the purified protein can be obtained. The purified protein may be recovered in a solution state containing the purified protein dissolved therein, or may be recovered in a freeze-dried state.

In one embodiment, the contact of the protein-containing solution with the polyketone is not particularly limited as long as the protein-containing solution can be contacted with the polyketone. Examples thereof include passing the protein-containing solution through the polyketone medium, and immersion of the polyketone medium in the protein-containing solution. It is preferred to pass the protein-containing solution through the polyketone medium from the viewpoint of relatively easily controlling the purification of the protein of interest.

In one embodiment, examples of the passage of the protein-containing solution through the polyketone medium include passing the protein-containing solution through the polyketone medium using a syringe or a pump. The method for passing the protein-containing solution through the polyketone medium can be any method by which the protein-containing solution injected toward a certain portion in the polyketone medium can pass through the polyketone medium so that the protein-containing solution is recovered from another portion of the polyketone medium. Aside from the protein-containing solution, a buffer solution may be passed through the polyketone medium before or after passage of the protein-containing solution through the polyketone medium. For the recovery of the protein-containing solution, the whole protein-containing solution passed through the polyketone medium may be recovered, or a fraction may be obtained on a constant volume basis. Fractions containing the purified protein can be collected and combined into one portion to recover the purified protein. The flow rate at which the protein-containing solution is passed through the polyketone medium is not particularly limited. An example of the lower limit value thereof is 0.1 mL/min or more per mL of the polyketone medium. In an alternative aspect, an example thereof is 0.5 mL/min or more. In an alternative aspect, an example thereof is 1.0 mL/min or more. In a further alternative aspect, an example thereof is 5 mL/min or more.

In one embodiment, examples of the immersion of the protein-containing solution in the polyketone medium include adding the polyketone medium into the protein-containing solution. In this respect, the protein-containing solution with the polyketone medium added therein may be shaken with a shaker or the like or stirred with a stirring bar or the like, or the solution may be left standing. The polyketone medium immersed can be removed from the protein-containing solution to recover the purified protein. Examples of the method for removing the polyketone medium include centrifugation and filtration.

### 2. Method for removing protein aggregate

The method for selectively removing a protein aggregate contained in a protein-containing solution from the protein-containing solution according to an embodiment comprises the step of: (b1) contacting the protein-containing solution with a medium comprising polyketone.

In one embodiment, the polyketone is not particularly limited as long as the polyketone mentioned above is used.

In one embodiment, the shape of the polyketone medium is not particularly limited as long as the shape mentioned above is used.

In the case where the polyketone medium is a porous molded body, the pore size of the polyketone medium is not particularly limited as long as it is the pore size mentioned above.

The pore size distribution of the polyketone medium may be three-dimensionally uniform or non-uniform. The three-dimensionally uniform membrane pore size can be confirmed from a three-dimensional SEM image or the like of the membrane.

In one embodiment, the protein to be purified is not particularly limited as long as it is the protein to be purified mentioned above. The molecular weight of the protein to be purified is not particularly limited as long as it is the molecular weight mentioned above.

In one embodiment, the protein-containing solution is not particularly limited as long as the protein-containing solution mentioned above is used.

In one embodiment, the concentration of the protein solution or the buffer solution is not particularly limited as long as it is the concentration mentioned above .

The pH of the protein solution or the buffer solution is not particularly limited as long as it is the pH mentioned above. The method for measuring the pH is not particularly limited as long as the method mentioned above is used.

The electrical conductivity of the protein solution or the buffer solution is not particularly limited as long as it is the electrical conductivity mentioned above. The method for measuring the electrical conductivity is not particularly limited as long as the method mentioned above is used.

In one embodiment, the protein aggregate is not particularly limited as long as it is the protein aggregate mentioned above.

In one embodiment, the contact of the protein-containing solution with the polyketone medium is not particularly limited as long as the contact method mentioned above is used. It is preferred to pass the protein-containing solution through the polyketone medium from the viewpoint of relatively easily controlling aggregate removal.

In one embodiment, the passage of the protein-containing solution through the polyketone medium is not particularly limited as long as the passage method mentioned above is used. As mentioned above, examples thereof include passing the protein-containing solution through the polyketone medium using a syringe or a pump. The method for passing the protein-containing solution through the polyketone medium can be any method by which the protein-containing solution injected toward a certain portion in the polyketone medium can pass through the polyketone medium so that the protein-containing solution is recovered from another portion of the polyketone medium. Aside from the protein-containing solution, a buffer solution may be passed through the polyketone medium before or after passage of the protein-containing solution through the polyketone medium. The flow rate at which the protein-containing solution is passed through the polyketone medium is not particularly limited. An example of the lower limit value thereof is 0.1 mL/min or more per mL of the polyketone medium. In an alternative aspect, an example thereof is 0.5 mL/min or more. In an alternative aspect, an example thereof is 1.0 mL/min or more. In a further alternative aspect, an example thereof is 5 mL/min or more.

In one embodiment, in the case where the polyketone medium is a membrane, the membrane volume is not particularly limited as long as it is the membrane volume mentioned above.

In one embodiment, the surface area of the polyketone medium is not particularly limited as long as it is the surface area mentioned above.

In one embodiment, examples of the immersion of the protein-containing solution in the polyketone medium include adding the polyketone medium into the protein-containing solution. In this respect, the protein-containing solution containing the polyketone medium may be shaken with a shaker or the like or stirred with a stirring bar or the like, or the solution may be left standing. The polyketone medium immersed can be removed from the protein-containing solution to recover the purified protein. Examples of the method for removing the polyketone carrier include centrifugation and filtration.

In one embodiment, the removal of the protein aggregate is not particularly limited as long as the contact of the protein-containing solution with the polyketone can selectively reduce a protein aggregate content from the content of the aggregate of the protein of interest in the protein-containing solution before the contact with the polyketone, as mentioned above. Examples of the contact method include passing the protein-containing solution through the polyketone medium, and immersion, shaking, stirring, and leaving standing the polyketone in the protein-containing solution. Examples of the selective reduction include decrease in the amount of the protein aggregate as compared with the amount of the monomer of the protein of interest.

### 3. Medium for protein aggregate removal

The medium for selectively removing a protein aggregate according to an embodiment is not particularly limited as long as the medium comprises polyketone. The medium substantially consists of polyketone.

In one embodiment, the polyketone is not particularly limited as long as the polyketone mentioned above is used.

In one embodiment, the shape of the polyketone medium is not particularly limited as long as it is the shape mentioned above.

In one embodiment, in the case where the polyketone medium is a membrane, the membrane volume is not particularly limited as long as it is the membrane volume mentioned above.

In one embodiment, the surface area of the polyketone medium is not particularly limited as long as it is the surface area mentioned above.

In the case where the polyketone medium is a porous body, the pore size of the polyketone medium is not particularly limited as long as it is the pore size mentioned above.

In one embodiment, the pore size distribution of the polyketone medium may be three-dimensionally uniform or non-uniform. The three-dimensionally uniform membrane pore size can be confirmed from a three-dimensional SEM image of the membrane.

In one embodiment, the protein to be purified is not particularly limited as long as the protein to be purified mentioned above is used. The molecular weight of the protein to be purified is not particularly limited as long as it is the molecular weight mentioned above.

In one embodiment, examples of the protein aggregate include, but are not particularly limited to, the protein aggregate mentioned above and other protein aggregates.

The medium comprising polyketone according to an embodiment can be used as described in the preceding section 1. or 2. As a result, the aggregate of the protein of interest can be efficiently removed.

### 4. Method for purifying protein

The method for purifying a protein according to an embodiment comprises the steps of: (a1) contacting a protein-containing solution with a porous medium comprising polyketone (hereinafter, also referred to as a "polyketone porous medium"); and (a2) passing the protein-containing solution contacted with the porous medium comprising polyketone through a virus removal membrane.

The method for purifying a protein according to an embodiment comprises the steps of: (β1) contacting a protein-containing solution with a porous medium having a specific surface area of 4.0 m²/mL or larger and a porosity of 50% or more; and (β2) passing the protein-containing solution contacted with the porous medium through a virus removal membrane.

In one embodiment, the polyketone is not particularly limited as long as the polyketone mentioned above is used.

In one embodiment, the shape of the porous medium is not particularly limited as long as the shape is the same as that of the polyketone medium mentioned above.

The pore size of the porous medium is not particularly limited as long as the pore size is the same as that of the polyketone medium mentioned above in the case where the polyketone medium is the porous molded body.

The pore size distribution of the porous medium may be three-dimensionally uniform or non-uniform. The three-dimensionally uniform membrane pore size can be confirmed from a three-dimensional SEM image of the membrane.

In one embodiment, the shape of the porous medium is not particularly limited as long as the shape permits protein purification. Examples thereof include shapes such as beads, gels, sponges, and powders. In an alternative aspect, an example thereof is a fiber or a membrane (flat membrane or hollow fiber membrane). In an alternative aspect, an example of the shape of the porous medium is a flat membrane or a hollow fiber membrane. In a further alternative aspect, an example thereof is a flat membrane. In a further alternative aspect, an example thereof is a hollow fiber membrane. The porous medium may be a porous form. The porous form may be a microporous membrane, a non-woven fabric, a woven fabric, a monolith, a gel, or a particle bed.

In one embodiment, examples of the raw material of the porous medium having a specific surface area of 4.0 m²/mL or larger and a porosity of 50% or more include polyketone and nylon. In an alternative aspect, an example thereof is polyketone. The polyketone can be produced by a method described in, for example, Patent Literature 4. The nylon can be produced by a method known in the art.

In one embodiment, in the case where the porous medium is a membrane, the membrane volume is not particularly limited as long as the membrane volume permits protein purification. An example of the lower limit value of the membrane volume is 0.10 mL or larger per 1.0 g of the protein to be purified. In an alternative aspect, an example thereof is 0.50 mL or larger. In an alternative aspect, an example thereof is 1.0 mL or larger. In a further alternative aspect, an example thereof is 1.5 mL or larger. An example of the upper limit value of the membrane volume is 100000 mL or smaller per 1.0 g of the protein to be purified. In an alternative aspect, an example thereof is 10000 mL or smaller. In an alternative aspect, an example thereof is 1000 mL or smaller. In an alternative aspect, an example thereof is 100 mL or smaller. In a further alternative aspect, an example thereof is 10 mL or smaller.

In one embodiment, the surface area of the porous medium is not particularly limited as long as the surface area permits protein purification. An example of the lower limit value of the surface area is 0.0001 m² or larger per 1.0 g of the protein to be purified. In an alternative aspect, an example thereof is 0.001 m² or larger. In an alternative aspect, an example thereof is 0.01 m² or larger. In an alternative aspect, an example thereof is 0.1 m² or larger. In an alternative aspect, an example thereof is 1.0 m² or larger. In a further alternative aspect, an example thereof is 5.0 m² or larger. An example of the upper limit value of the surface area is 1000000 m² or smaller per 1.0 g of the protein to be purified. In an alternative aspect, an example thereof is 100000 m² or smaller. In an alternative aspect, an example thereof is 10000 m² or smaller. In an alternative aspect, an example thereof is 1000 m² or smaller. In an alternative aspect, an example thereof is 100 m² or smaller. In a further alternative aspect, an example thereof is 30 m² or smaller. The surface area is calculated from a specific surface area. The specific surface area means the surface area of the polyketone per unit volume of the polyketone medium. The surface area is calculated from the specific surface area and is the product of the specific surface area and the volume of the polyketone medium.

In one embodiment, examples of the specific surface area of the porous medium per unit volume of the porous medium include 4.0 m²/mL or larger. In a further alternative aspect, an example thereof is 5.0 m²/mL or larger. In a further alternative aspect, an example thereof is 6.0 m²/mL or larger. In a further alternative aspect, an example thereof is 7.0 m²/mL or larger. In a further alternative aspect, an example thereof is 8.0 m²/mL or larger. In a further alternative aspect, an example thereof is 9.0 m²/mL or larger. In a further alternative aspect, an example thereof is 10.0 m²/mL or larger. In a further alternative aspect, an example thereof is 11.0 m²/mL or larger. In a further alternative aspect, an example thereof is 12.0 m²/mL or larger. Further examples of the specific surface area of the porous medium include 50 m²/mL or smaller. In a further alternative aspect, an example thereof is 49.0 m²/mL or larger. In a further alternative aspect, an example thereof is 48.0 m²/mL or larger. In a further alternative aspect, an example thereof is 47.0 m²/mL or larger. In a further alternative aspect, an example thereof is 46.0 m²/mL or larger. In a further alternative aspect, an example thereof is 45.0 m²/mL or larger. In a further alternative aspect, an example thereof is 44.0 m²/mL or larger. In a further alternative aspect, an example thereof is 43.0 m²/mL or larger. In a further alternative aspect, an example thereof is 42.0 m²/mL or larger. In a further alternative aspect, an example thereof is 41.0 m²/mL or larger. In a further alternative aspect, an example thereof is 40.0 m²/mL or larger. In a further alternative aspect, an example thereof is 39.0 m²/mL or larger. In a further alternative aspect, an example thereof is 38.0 m²/mL or larger. In a further alternative aspect, an example thereof is 37.0 m²/mL or larger. In a further alternative aspect, an example thereof is 36.0 m²/mL or larger. In a further alternative aspect, an example thereof is 35.0 m²/mL or larger. In a further alternative aspect, an example thereof is 34.0 m²/mL or larger. In a further alternative aspect, an example thereof is 33.0 m²/mL or larger. In a further alternative aspect, an example thereof is 32.0 m²/mL or larger. In a further alternative aspect, an example thereof is 31.0 m²/mL or larger. In a further alternative aspect, an example thereof is 30.0 m²/mL or larger. In a further alternative aspect, an example thereof is 29.0 m²/mL or larger. In a further alternative aspect, an example thereof is 28.0 m²/mL or larger. In a further alternative aspect, an example thereof is 27.0 m²/mL or larger. In a further alternative aspect, an example thereof is 26.0 m²/mL or larger. In a further alternative aspect, an example thereof is 25 m²/mL or smaller. In a further alternative aspect, an example thereof is 24 m²/mL or smaller. In a further alternative aspect, an example thereof is 23 m²/mL or smaller. In a further alternative aspect, an example thereof is 22 m²/mL or smaller. In a further alternative aspect, an example thereof is 21 m²/mL or smaller.

Examples of the porosity of the porous medium having the specific surface area include 50% or more. In a further alternative aspect, an example thereof is 55% or more. In a further alternative aspect, an example thereof is 60% or more. In a further alternative aspect, an example thereof is 65% or more. In a further alternative aspect, an example thereof is 68% or more. In a further alternative aspect, an example thereof is 69% or more. In a further alternative aspect, an example thereof is 70% or more. In a further alternative aspect, an example thereof is 71% or more. In a further alternative aspect, an example thereof is 72% or more. In a further alternative aspect, an example thereof is 73% or more. In a further alternative aspect, an example thereof is 74% or more. In a further alternative aspect, an example thereof is 75% or more. In a further alternative aspect, an example thereof is 76% or more. Further examples of the porosity of the porous medium include 99% or less. In a further alternative aspect, an example thereof is 95% or less. In a further alternative aspect, an example thereof is 90% or less.

In one embodiment, the protein to be purified is not particularly limited as long as the protein comprises a substance (biopolymer) causative of the fouling of a virus removal membrane when applied directly to the virus removal membrane and the fouling of the virus removal membrane is reduced as a result of contacting the protein with the porous medium. Examples of the protein to be purified include albumin, globulin, and fibrinogen. The antibody is not particularly limited as long as the antibody mentioned above is used. Examples thereof include monoclonal antibodies and polyclonal antibodies including any of IgM, IgD, IgG, IgA, and IgE. The antibody may be derived from a plasma product or may be derived from a cell culture solution, for example. In the case of obtaining the antibody by cell culture, animal cells or a microbe can be used as cells. The animal cells are not particularly limited by their type. Examples thereof include CHO cells, Sp2/0 cells, NS0 cells, Vero cells, and PER.C6 cells. The microbe is not particularly limited by its type. Examples thereof include *E. coli* and yeasts. 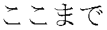

In one embodiment, the molecular weight of the protein is not particularly limited as long as the protein comprises a substance (biopolymer) causative of the fouling of a virus removal membrane and causes fouling of the virus removal membrane and the fouling of the virus removal membrane is reduced as a result of contacting the protein with the porous medium. An example of the lower limit value of the molecular weight is 0.1 kDa or higher. In an alternative aspect, an example thereof is 1 kDa or higher. In an alternative aspect, an example thereof is 10 kDa or higher. In an alternative aspect, an example thereof is 50 kDa or higher. In a further alternative aspect, an example thereof is 100 kDa or higher. An example of the upper limit value of the molecular weight is 10000 kDa or lower. In an alternative aspect, an example thereof is 5000 kDa or lower. In an alternative aspect, an example thereof is 1000 kDa or lower. In an alternative aspect, an example thereof is 500 kDa or lower. In a further alternative aspect, an example thereof is 200 kDa or lower.

In one embodiment, the pH of the protein solution or the buffer solution is not particularly limited as long as the pH allows the protein of interest and a substance (biopolymer) causative of the fouling of a virus removal membrane to be separated with the porous medium. An example of the lower limit value of the pH is 4.0 or higher. In an alternative aspect, an example thereof is 4.5 or higher. In a further alternative aspect, an example thereof is 5.0 or higher. In a further alternative aspect, an example thereof is 5.5 or higher. In a further alternative aspect, an example thereof is 6.0 or higher. An example of the upper limit value of the pH is 10.0 or lower. In an alternative aspect, an example thereof is 9.0 or lower. In a further alternative aspect, an example thereof is 8.0 or lower. In a further alternative aspect, an example thereof is 8.5 or lower. In a further alternative aspect, an example thereof is 8.0 or lower. In a further alternative aspect, an example thereof is 7.5 or lower. In a further alternative aspect, an example thereof is 7.0 or lower. Examples of the method for measuring the pH include, but are not particularly limited to, methods using hydrogen electrodes, quinhydrone electrodes, antimony electrodes, or glass electrodes. Among them, a method using a glass electrode is preferred.

The electrical conductivity of the protein solution or the buffer solution is not particularly limited as long as the electrical conductivity allows a monomer of the protein of interest and a substance (biopolymer) causative of the fouling of a virus removal membrane to be separated with the porous medium. An example of the lower limit value of the electrical conductivity is 0 mS/cm or more. In an alternative aspect, an example thereof is 1 mS/cm or more. In an alternative aspect, an example thereof is 2 mS/cm or more. In an alternative aspect, an example thereof is 3 mS/cm or more. In an alternative aspect, an example thereof is 4 mS/cm or more. In a further alternative aspect, an example thereof is 5 mS/cm or more. An example of the upper limit value of the electrical conductivity is 100 mS/cm or less. In an alternative aspect, an example thereof is 90 mS/cm or less. In a further alternative aspect, an example thereof is 80 mS/cm or less. In a further alternative aspect, an example thereof is 70 mS/cm or less. In a further alternative aspect, an example thereof is 60 mS/cm or less. In a further alternative aspect, an example thereof is 50 mS/cm or less. In a further alternative aspect, an example thereof is 40 mS/cm or less. The method for measuring the electrical conductivity is not particularly limited. For example, a method stipulated in JIS K0130 "General rules for electrical conductivity measuring method " is available. Examples of such a method include an alternating current two-electrode scheme, an alternating current four-electrode scheme, and an electromagnetic induction scheme. Among them, an alternating current two-electrode scheme is preferred.

In one embodiment, the purified protein is not particularly limited as long as the purified protein is a protein with the content of a substance (biopolymer) causative of the fouling of virus removal membrane reduced from the content of the substance (biopolymer) causative of the fouling of virus removal membrane in proteins in the protein-containing solution before purification, i.e., before contact with the porous medium.

In one embodiment, the contact of the protein-containing solution with the porous medium is not particularly limited as long as the protein-containing solution can be contacted with the porous medium. Examples thereof include passing the protein-containing solution through the porous medium, and immersion of the porous medium in the protein-containing solution. It is preferred to pass the protein-containing solution through the porous medium from the viewpoint of relatively easily controlling the purification of the protein of interest.

In one embodiment, examples of the passage of the protein-containing solution through the porous medium include passing the protein-containing solution through the porous medium using a syringe or a pump. The method for passing the protein-containing solution through the porous medium can be any method by which the protein-containing solution injected toward a certain portion in the porous medium can pass through the porous medium so that the protein-containing solution is recovered from another portion of the porous medium. Aside from the protein-containing solution, a buffer solution may be passed through the porous medium before or after passage of the protein-containing solution through the porous medium. For the recovery of the protein-containing solution, the whole protein-containing solution passed through the porous medium may be recovered, or a fraction may be obtained on a constant volume basis. Fractions containing the purified protein can be collected and combined into one portion to recover the purified protein. The flow rate at which the protein-containing solution is passed through the porous medium is not particularly limited. An example of the lower limit value thereof is 0.1 mL/min or more per mL of the polyketone medium. In an alternative aspect, an example thereof is 0.5 mL/min or more. In an alternative aspect, an example thereof is 1.0 mL/min or more. In a further alternative aspect, an example thereof is 5 mL/min or more.

In one embodiment, examples of the immersion of the protein-containing solution in the porous medium include adding the porous medium into the protein-containing solution. In this respect, the protein-containing solution with the porous medium added therein may be shaken with a shaker or the like or stirred with a stirring bar or the like, or the solution may be left standing. The porous medium immersed can be removed from the protein-containing solution to recover the purified protein. Examples of the method for removing the porous medium include centrifugation and filtration.

In one embodiment, the protein to be purified is not particularly limited as long as the protein to be purified mentioned above is used. The molecular weight of the protein to be purified is not particularly limited as long as the molecular weight mentioned above is used.

In one embodiment, the proteins before filtration through the porous medium may include a biopolymer, in addition to a monomer of the protein of interest. The biopolymer is not particularly limited as long as the biopolymer is a substance causative of the fouling of a virus removal filter. An example thereof is an aggregate of the protein of interest. In an alternative aspect, an example thereof is an aggregate of the protein of interest and a protein other than the protein of interest. In one embodiment, examples of the protein aggregate include aggregates formed by aggregating a plurality of monomers of the protein.

In one embodiment, the protein-containing solution is not particularly limited as long as the protein-containing solution mentioned above is used.

In one embodiment, the concentration of the protein solution or the buffer solution is not particularly limited as long as it is the concentration mentioned above.

The pH of the protein solution or the buffer solution is not particularly limited as long as the pH mentioned above is used. The method for measuring the pH is not particularly limited as long as it is the method mentioned above.

The electrical conductivity of the protein solution or the buffer solution is not particularly limited as long as it is the electrical conductivity mentioned above. The method for measuring the electrical conductivity is not particularly limited as long as it is the method mentioned above.

In one embodiment, the protein aggregate is not particularly limited as long as it is the protein aggregate mentioned above.

In one embodiment, the virus removal membrane is not particularly limited as long as the virus removal membrane can substantially remove a virus. As an example, the virus removal membrane has a nominal pore size of approximately 15 nm or larger and approximately 75 nm or smaller. For example, a membrane having a nominal pore size of 20 nm is capable of removing, for example, parvovirus (diameter: 18 nm or larger and 26 nm or smaller) while permitting passage of a protein having a size of 160 kD (approximately 8 nm), for example, a monoclonal antibody. For example, retrovirus (diameter: 80 nm or larger and 100 nm or smaller) can be removed using, for example, a membrane having a nominal pore size of 35 nm or larger.

In one embodiment, the virus removal membrane has a molecular weight cut-off of 100 kD to 1000 kD. In relation to this, the molecular weight cut-off (MWCO) of the membrane refers to the nominal molecular weight of molecules and particles, 90% of which can pass through the membrane.

In one embodiment, the raw material of the virus removal membrane is not particularly limited as long as the membrane can remove a virus. Examples thereof include polyethersulfone, polysulfone, polyvinylidene fluoride, cellulose, cellulose derivatives and mixtures thereof. Each of such membranes can be produced by a method known in the art (see Patent Literatures 6 and 7 or Non Patent Literatures 2 to 5).

In one embodiment, in the case of connecting the polyketone porous medium used in the step (a1) or the porous medium used in the step (b1) with the virus removal membrane used in the step (a2) or the step (b2), these membranes may be connected directly, or another filtration step may intervene therebetween.

In one embodiment, in the case of connecting the polyketone porous medium used in the step (a1) or the porous medium used in the step (b1) with the virus removal membrane used in the step (a2) or the step (b2), examples of the upper limit of the pressure applied to the polyketone porous medium used in the step (a1) or the porous medium used in the step (b1) include 1/2 or less of the pressure applied to the virus removal membrane. In an alternative aspect, an example thereof is 1/2.5 or less. In an alternative aspect, an example thereof is 1/3 or less. In an alternative aspect, an example thereof is 1/3.5 or less. In an alternative aspect, an example thereof is 1/4 or less. Examples of the lower limit thereof include 1/40 or more. In an alternative aspect, an example thereof is 1/35 or more. In an alternative aspect, an example thereof is 1/30 or more. In an alternative aspect, an example thereof is 1/25 or more. In an alternative aspect, an example thereof is 1/20 or more.

In one embodiment, examples of the amount of the protein per arbitrary time treated with the virus removal membrane after contact with the porous medium include 1.05 or more times the amount of the protein per arbitrary time treated with the virus removal membrane without contact with the porous medium. In an alternative aspect, an example thereof is 1.1 or more times. In an alternative aspect, an example thereof is 1.2 or more times. In an alternative aspect, an example thereof is 1.5 or more times. In an alternative aspect, an example thereof is 2.0 or more times. In an alternative aspect, an example thereof is 2.5 or more times. In an alternative aspect, an example thereof is 3.0 or more times. In an alternative aspect, an example thereof is 3.5 or more times. In an alternative aspect, an example thereof is 4.0 or more times. In an alternative aspect, an example thereof is 4.5 or more times. In an alternative aspect, an example thereof is 5.0 or more times.

The virus removal rate of the protein-containing solution treated with the virus removal membrane is at least 99.9%.

In one embodiment, the prefilter is a membrane installed before the virus removal membrane, and can improve the throughput or flux of the protein of interest by reducing a substance causative of the fouling of the virus removal membrane contained in the protein-containing solution that is be passed through the virus removal membrane.

### Examples

Hereinafter, examples of the embodiments will be described. However, these examples do not limit the embodiments by any means.

### [Example A1]

### (1) Preparation of porous medium comprising polyketone

Polyketone porous membrane PK1 having the characteristics described in Table 1 was prepared as the porous medium comprising polyketone by the method described in the paragraphs [0062] to [0071] of Patent Literature 4.

### (2) Specific surface area of polyketone porous membrane

The specific surface area of the polyketone porous membrane PK1 to be evaluated was measured by a BET specific surface area measurement instrument (Macsorb HM model-1201, manufactured by Mountech Co., Ltd.). The results are shown in Table 1.

### (3) Preparation of antibody solution

A culture solution containing CRL12445 antibody-producing cells was filtered using a filtration membrane (manufactured by Asahi Kasei Medical Co., Ltd., trade name BioOptimal(R) MF-SL) to obtain an antibody solution (culture supernatant) containing impurities and a 0.74 g/L monoclonal antibody.

### (4) Preparation of antibody protein monomer solution

According to a commercially typical method, the antibody solution obtained in the section (3) was purified by chromatography such as protein A affinity chromatography or ion-exchange chromatography, and the buffer was replaced with a 15 mmol/L tris-HCl buffer solution (pH 7.0, 5 mS/cm) containing sodium chloride to prepare an antibody protein monomer solution. The electrical conductivity of the solution was measured by electrical conductivity meter CM-40S (manufactured by DKK-TOA Corp.).

### (5) Preparation of antibody protein aggregate solution

The pH of a portion of the antibody protein monomer solution obtained in the section (4) was adjusted to 2.5 by the addition of hydrochloric acid and kept for one hour. Then, the solution was neutralized using an sodium hydroxide aqueous solution, and the buffer was replaced with a 15 mmol/L tris-HCl buffer solution (pH 7.0, 5 mS/cm) containing sodium chloride to prepare an antibody protein aggregate solution rich in antibody protein aggregates.

### (6) Preparation of antibody solution containing aggregate

The antibody protein monomer solution obtained in the section (4) and the antibody protein aggregate solution obtained in the section (5) were mixed to prepare a solution containing 4% antibody protein aggregates. In this context, the term "4%" refers to the proportion of a dimer and higher multimeric aggregates in the antibody proteins contained in the solution. As described in the section (7), the amounts of the aggregates were measured by size exclusion chromatography.

### (7) Measurement of amount of aggregate

The amounts of the aggregates were measured for the solution containing the antibody protein aggregates prepared in the section (6) using a size exclusion chromatography (SEC) apparatus under the following conditions.
Column: ACQUITY UPLC BEH200 SEC 1.7 mm (manufactured by Waters Corp.)
Column temperature: 30°C
System: ACQUITY UPLC H CLASS (manufactured by Waters Corp.)
Mobile phase: 0.1 mol/L disodium hydrogen phosphate + 0.2 mol/L L(+)-arginine aqueous solution (adjusted to pH 6.7 with hydrochloric acid)

### (8) Preparation of polyketone porous membrane module

The polyketone porous membrane PK1 obtained in the section (1) was cut into a round shape having a diameter of 2.5 cm. Three sheets thereof were layered and in this state, sandwiched in a syringe holder (Advantec KS-25 stainless steel syringe holder) with an effective membrane area of 3.8 cm² to prepare a polyketone porous membrane module according to Example A1. The membrane volume used of the polyketone porous membrane PK1 in the polyketone porous membrane module according to Example A1 is shown in Table 1.

### (9) Preparation of virus removal membrane

A hollow fiber membrane prepared by the method described in the paragraphs [0066] to [0088] of Patent Literature 10 was assembled with an effective membrane area of 2.8 cm². The resulting filter was used as a virus removal membrane. The effective membrane area was calculated from the inner diameter and effective length of the hollow fiber membrane.

### (10) Measurement of pressure ratio

A liquid pressure gauge was installed between the polyketone porous membrane module according to Example A1 prepared in the section (8) and the virus removal membrane and in this state, connected. Water was passed therethrough at a pressure of 196 kPa, and the pressure (kPa) applied to the virus removal membrane was measured. The value of the pressure applied to the virus removal membrane was subtracted from 196 kPa. The obtained value was regarded as a pressure applied to the polyketone porous membrane. A numerical value obtained by dividing the pressure applied to the polyketone porous membrane by the pressure applied to the virus removal membrane is described as a pressure ratio in Table 1.

### (11) Measurement of antibody throughput 1

The polyketone porous membrane module according to Example A1 prepared in the section (8) and the virus removal membrane obtained in the section (9) were connected. The antibody solution containing the aggregates prepared in the section (6) was passed therethrough at a constant pressure of 196 kPa in a dead-end mode. In this respect, the amount of the solution filtered until when an antibody solution throughput speed per unit time per unit area of the virus removal membrane (LMH = solution throughput (L) / virus removal membrane area (m²)/ hour) fell below 20 LMH was regarded as a solution throughput (L). The antibody throughput was calculated from the product of the concentration and solution throughput of the obtained antibody solution. A value obtained by dividing the antibody throughput by the virus removal membrane area used is described as antibody throughput 1 in Table 1.

### (12) Measurement of antibody throughput 2

The amount of improvement in the throughput of the virus removal membrane (kg) by using the polyketone porous membrane module according to Example A1 over the throughput of treatment with only the virus removal membrane without the use of the polyketone porous membrane module according to Example A1 per unit volume (L) of the polyketone porous membrane module according to Example A1, was calculated as antibody throughput 2. First, in the same way as in the section (11), the amount of the solution filtered until when an antibody solution throughput speed per unit time per unit area of the virus removal membrane (LMH = solution throughput (L) / virus removal membrane area (m²)/ hour) fell below 20 LMH was regarded as a solution throughput (L). The antibody throughput (kg/m²) was calculated from the product of the concentration and solution throughput of the obtained antibody solution. The amount of the antibody treatable with only the virus removal membrane without the use of the polyketone porous membrane module according to Example A1 was subtracted from the calculated value and divided by the membrane volume used (L) described in Table 1. The obtained value was regarded as antibody throughput 2 (kg/L) and is described in Table 1. As is evident from the results, the polyketone porous membrane module according to Example A1 had a large specific surface area and therefore drastically improved the antibody throughput of the virus removal membrane.

### [Example A2]

The same operation as in Example A1 was performed except that a polyketone porous membrane module according to Example A2 prepared using polyketone porous membrane PK2 having the characteristics described in Table 1 was used. The results are shown in Table 1. As shown in the results, since PK2 had a larger pore size than that of PK1, the specific surface area was decreased and the amount of improvement in the antibody throughput was slightly decreased but the pressure loss was further decreased.

### [Example A3]

The same operation as in Example A1 was performed except that a polyketone porous membrane module according to Example A3 which consisted of polyketone porous hollow fiber membrane (inner diameter: 520 mm, outer diameter: 840 mm) PK3 having the characteristics described in Table 1 and had an effective membrane volume of 0.12 mL was prepared by the method described in the paragraphs [0074] to [0077] of Patent Literature 4. The results are shown in Table 1. As is evident from the results, the polyketone porous membrane module according to Example A3 had a large specific surface area and therefore exhibited a high antibody throughput, while suppressing pressure loss owing to the hollow fiber structure.

### [Comparative Example A1]

The same operation as in Example A1 was performed except that a virus removal membrane falling under the step (a2) or the step (b2) was used without carrying out a step corresponding to the step (a1) or (b1). The results are shown in Table 1. As is evident from the results, the antibody throughput for the virus removal membrane was small, as compared with Examples A1 to A3.

### [Comparative Example A2]

The same operation as in Example A1 was performed except that nylon 1 (nylon membrane (Whatman(R): 7402-002), manufactured by GE Healthcare Japan Corp., pore size: 0.2 mm, membrane volume: 0.12 mL) was used in a step corresponding to the step (a1) or the step (b1). The results are shown in Table 1. As is evident from the results, because of difference in material for the medium or because of a small specific surface area, the amount of improvement in the throughput per unit volume of the porous medium was small in terms of the antibody throughput for the virus removal membrane, as compared with Examples A1 to A3.

### [Comparative Example A3]

The same operation as in Example A1 was performed except that nylon 2 (nylon membrane (Acrodisc(R) Syringe Filter: PN4906), manufactured by Pall Corp., pore size: 0.2 mm, membrane volume: 0.04 mL) was used in a step corresponding to the step (a1) or the step (b1). The results are shown in Table 1. As is evident from the results, because of difference in material for the medium or because of a small specific surface area, the amount of improvement in the throughput per unit volume of the porous medium was small in terms of the antibody throughput for the virus removal membrane, as compared with Examples A1 to A3.

**[Table 1]**

| | | Example A1 | Example A2 | Example A3 | Comparat ive Example A1 | Comparat ive Example A2 | Comparat ive Example A3 |
|---|---|---|---|---|---|---|---|
| Porous medium in step (a1) or (b1) | Type of membrane | PK1 | PK2 | PK3 | - | Nylon 1 | Nylon 2 |
| | Shape | Flat membran e | Flat membran e | Hollow fiber | - | Flat membrane | Flat membrane |
| | Specific surface area (m2/mL) | 15 | 8.1 | 12 | - | 3.9 | 3.3 |
| | Membrane volume (mL) | 0.11 | 0.11 | 0.12 | | 0.12 | 0.04 |
| | Porosity (%) | 77 | 79 | 78 | - | 67 | 64 |
| | Pore size (um) | 0.1 | 0.2 | 0.1 | - | 0.2 | 0.2 |
| | Membrane thickness (um) | 300 | 297 | 160 | | 320 | 130 |
| | Effective membrane area (cm2) | 3.8 | 3.8 | 5.7 | - | 3.8 | 3.14 |
| Antibody throughput 1 (kg/m2) (Throughput per unit area of virus removal membrane) | | 0.53 | 0.39 | 0.47 | 0.094 | 0.32 | 0.12 |
| Antibody throughput 2(kg/L) (Amount of throughput per porous medium volume) | | 1.15 | 0.79 | 0.94 | - | 0.56 | 0.19 |
| Pressure applied in step (a1) or (b1) / pressure applied in step (a2) or (b2) | | 1/4.9 | 1/17 | 1/19 | - | 1/17 | 1/32 |

### [Example B1]

### (1) Preparation of medium comprising polyketone

Polyketone membrane 1 (pore size: 0.2 mm) having the characteristics described in Table 2 was prepared as the medium comprising polyketone by the method described in the paragraphs [0062] to [0071] of Patent Literature 4. The prepared polyketone membrane 1 was cut into a round shape having a diameter of 2.5 cm. The membrane thickness was measured by Digimatic Indicator ID-C112XBS (manufactured by Mitutoyo Corp.) and determined to be 100 mm. Two sheets of the cut membrane were loaded in stainless steel holder KS-25 (manufactured by Advantech Co., Ltd., effective membrane area: 3.8 cm²). The effective membrane volume of the polyketone membrane in the holder was calculated to be 0.08 mL.

### (2) Surface area of polyketone membrane

The specific surface area of the polyketone membrane 1 was measured by a BET specific surface area measurement instrument (Macsorb HM model-1201, manufactured by Mountech Co., Ltd.). From the measurement results, the surface area of the polyketone membrane 1 was calculated to be 0.65 m².

### (3) Preparation of antibody solution

A culture solution containing CRL12445 antibody-producing cells was filtered using a filtration membrane (manufactured by Asahi Kasei Medical Co., Ltd., trade name BioOptimal(R) MF-SL) to obtain an antibody solution (culture supernatant) containing impurities and a 0.74 g/L monoclonal antibody.

### (4) Purification of antibody protein with affinity column

The antibody solution prepared in the section (3) was added to a protein A column (manufactured by GE Healthcare Bio-Sciences AB, a column packed with MabSelect Sure) equilibrated with a phosphate buffer solution (20 mmol/L sodium phosphate + 150 mmol/L NaCl (pH 8.0)) so that the antibody protein was adsorbed to protein A. Next, a phosphate buffer solution (20 mmol/L sodium phosphate + 150 mmol/L NaCl (pH 8.0)) was passed through the column for washing. Then, an elution buffer solution (100 mmol/L sodium citrate (pH 3.6)) was passed through the column for the elution of antibody proteins from the protein A column to recover an antibody-containing solution with the level of impurities reduced to some extent. As a result of measuring the antibody proteins by the method described in the section (8) given below, only an antibody protein peak as a monomer (peak (3) in the section (8) given below) was confirmed whereas a peak of aggregate (1) or aggregate (2) was not able to be confirmed. The pH of the buffer solutions was measured using pH meter HM-30R (manufactured by DKK-TOA Corp.). The pH measurement of the buffer solutions given below was performed in the same way.

### (5) Preparation of aggregate

The pH of a portion of the antibody solution obtained in the section (4) was adjusted to 2.5 by the addition of hydrochloric acid and kept for one hour. Then, the solution was neutralized using a sodium hydroxide aqueous solution to prepare an antibody solution rich in antibody aggregates.

### (6) Preparation of antibody solution containing aggregate

A solution obtained by the buffer replacement of the antibody solution obtained in the section (4) with a 15 mmol/L tris-HCl buffer solution (pH 7.0, 5 mS/cm) containing an arbitrary amount of sodium chloride, and a solution obtained by the buffer replacement of the antibody solution rich in aggregates obtained in the section (5) with a 15 mmol/L tris-HCl buffer solution (pH 7.0, 5 mS/cm) containing an arbitrary amount of sodium chloride were mixed at an arbitrary ratio to prepare an antibody solution (hereinafter, also abbreviated to "SM") containing antibody protein aggregates. The electrical conductivity of the antibody solution was measured by electrical conductivity meter CM-40S (manufactured by DKK-TOA Corp.).

### (7) Passage of antibody-containing solution through polyketone membrane

The antibody solution containing antibody aggregate components (impurities) and a monomer component (physiologically active substance of interest), prepared in the section (6) was passed through the polyketone membrane 1 loaded in the holder in the section (1). For the passage of the antibody solution, AKTA purifier (manufactured by GE Healthcare Japan Corp.) was used, and a fraction was recovered with each volume of 1/10 of the total amount of the passed antibody solution. The amount of the solution added was 9 mL (concentration: 5.10 mg/mL, total amount of the antibody proteins: 45.9 mg), and the flow rate was 0.45 mL/min.

### (8) Measurement of amount of aggregate

Proteins contained in the antibody solution containing the aggregates, prepared in the section (6), and each fraction recovered in the section (7) were measured using a size exclusion chromatography (SEC) apparatus under the following conditions.
Column: ACQUITY UPLC BEH200 SEC 1.7 mm (manufactured by Waters Corp.)
Column temperature: 30°C
System: ACQUITY UPLC H CLASS (manufactured by Waters Corp.)
Mobile phase: 0.1 mol/L disodium hydrogen phosphate + 0.2 mol/L L(+)-arginine aqueous solution (adjusted to pH 6.7 with hydrochloric acid)

As a result of measuring the last fraction obtained in the section (7), the chromatography chart illustrated in Figure 1 was obtained. An enlarged view of Figure 1 is Figure 2. Since most of the proteins contained in the solution were derived from the antibody produced by the CHO cells CRL12445, it can be determined that antibody aggregates appear at the peaks (1) and (2) of Figure 2 while a monomer appears at the peak (3). The proportion of the aggregate (1) calculated from the area of the peak in the chromatography chart was 1.18%, the proportion of the aggregate (2) was 1.83%, and the proportion of the monomer was 96.99%. Likewise, this operation was also performed on an antibody solution containing other fractions and antibody protein aggregates to measure the proportions of aggregates and a monomer. In Examples and Comparative Examples given below, the peak of an aggregate appearing first is referred to as aggregate (1), and the peak of an aggregate appearing next is referred to as aggregate (2).

### (9) Evaluation of aggregate removal performance

The antibody concentration of each fraction was measured by a spectrophotometer (SpectraMax Plus 384, manufactured by Molecular Devices Japan). The content weights of aggregates and monomers contained in the antibody solution SM containing antibody protein aggregates and each of fractions FT1 to FT10 were calculated from the antibody concentration and the proportions of aggregates and a monomer measured in the section (8). The results are shown in Figure 3.

The chart shown in Figure 4 was obtained from the content weights of aggregates and a monomer in each fraction. M/V on the abscissa is a value obtained by dividing the accumulated weight of antibodies passed through the evaluation subject by the membrane volume of the evaluation subject. C/C0 on the ordinate is a value indicating the ratio of the concentration of an aggregate or a monomer contained in each fraction to that in the initially prepared antibody solution containing aggregates. C on the ordinate represents the concentration of the monomer or the aggregate (1) or (2) in each fraction, and C0 represents the concentration of the monomer or the aggregate (1) or (2) before passage through the evaluation subject.

In Figure 4, particularly, the proportions of the aggregate (1) and the aggregate (2) compared with the monomer were decreased in fractions having M/V of 400 mg/mL or less. It was therefore confirmed that the antibody aggregates were removed under this condition.

### [Example B2]

The same operation as in Example B1 was performed except that only the buffer solution was changed. Acetic acid and sodium acetate were mixed to prepare an acetate buffer solution (15 mmol/L acetate buffer solution, pH 5.5, electrical conductivity: 15 mS/cm). An antibody solution (antibody concentration: 5.06 mg/mL) obtained by buffer replacement with the acetate buffer solution was passed through the polyketone membrane 1. Then, the aggregate removal performance was evaluated by the same method as in the section (9) of Example B1. The results are shown in Figure 5. In Figure 5, particularly, the proportions of the aggregate (1) and the aggregate (2) compared with the monomer were decreased in fractions having M/V of 400 mg/mL or less. It was therefore confirmed that the antibody aggregates were removed under this condition.

### [Example B3]

The same operation as in Example B1 was performed except that only the pore size of the membrane was changed. 10 mL of the antibody solution (concentration: 5.12 mg/mL, total amount of the antibody proteins: 51.2 mg) was passed through polyketone membrane 2 (pore size: 0.1 mm, membrane thickness: 112 mm, 2 sheets layered, effective membrane volume: 0.09 mL, surface area: 1.37 m²) at a flow rate of 0.50 mL/min. The aggregate removal performance was evaluated by the same method as in the section (9) of Example B1. The results are shown in Figure 6. In Figure 6, particularly, the proportions of the aggregate (1) and the aggregate (2) compared with the monomer were decreased in fractions having M/V of 500 mg/mL or less. It was therefore confirmed that the antibody aggregates were removed under this condition.

### [Example B4]

The same operation as in Example B1 was performed except that only the pore size of the membrane was changed. 12 mL of the antibody solution (concentration: 5.08 mg/mL, total amount of the antibody proteins: 61.0 mg) was passed through polyketone membrane 3 (pore size: 0.4 mm, membrane thickness: 85 mm, 3 sheets layered, membrane volume: 0.10 mL, surface area: 0.40 m²) at a flow rate of 0.60 mL/min. The aggregate removal performance was evaluated by the same method as in the section (9) of Example B1. The results are shown in Figure 7. In Figure 7, particularly, the proportions of the aggregate (1) and the aggregate (2) compared with the monomer were decreased in fractions having M/V of 300 mg/mL or less. It was therefore confirmed that the antibody aggregates were removed under this condition.

### [Example B5]

The same operation as in Example B1 was performed except that only the electrical conductivity of the buffer solution was changed. 9 mL of the antibody solution (antibody concentration: 5.12 mg/mL, 15 mmol/L tris-HCl buffer solution, pH 7.0, electrical conductivity: 35 mS/cm) was passed through the polyketone membrane 1 at a flow rate of 0.45 mL/min. The aggregate removal performance was evaluated by the same method as in the section (9) of Example B1. The results are shown in Figure 8. In Figure 8, particularly, the proportions of the aggregate (1) and the aggregate (2) compared with the monomer were decreased in fractions having M/V of 500 mg/mL or less. It was therefore confirmed that the antibody aggregates were removed under this condition.

### [Example B6]

The same operation as in Example B1 was performed except that the type of the antibody and the type of the membrane were changed. 10 mL of a solution containing human monoclonal antibody AE6F4 (antibody concentration: 4.52 mg/mL) was passed through polyketone membrane 4 (pore size: 0.1 mm, membrane thickness: 101 mm, 2 sheets layered, membrane volume: 0.08 mL, surface area: 0.94 m²) at a flow rate of 0.50 mL/min. The aggregate removal performance was evaluated. The results are shown in Figure 9. In Figure 9, particularly, the proportions of the aggregate (1) and the aggregate (2) compared with the monomer were decreased in fractions having M/V of 400 mg/mL or less. It was therefore confirmed that the antibody aggregates were removed under this condition.

### [Example B7]

The same operation as in Example B3 was performed except that only the flow rate of passage was changed. 10 mL of the antibody solution (antibody concentration: 5.07 mg/mL) was passed through the polyketone membrane 2 at a flow rate of 3.0 mL/min. The aggregate removal performance was evaluated. The results are shown in Figure 10. In Figure 10, particularly, the proportions of the aggregate (1) and the aggregate (2) compared with the monomer were decreased in fractions having M/V of 500 mg/mL or less. It was therefore confirmed that the antibody aggregates were removed under this condition.

### [Example B8]

The same operation as in Example B3 was performed except that only the antibody concentration of the solution to be passed was changed. 10 mL of the antibody solution (antibody concentration: 14.57 mg/mL) was passed through the polyketone membrane 2 at a flow rate of 0.50 mL/min. The aggregate removal performance was evaluated. The results are shown in Figure 11. In Figure 11, particularly, the proportions of the aggregate (1) and the aggregate (2) compared with the monomer were decreased in fractions having M/V of 500 mg/mL or less. It was therefore confirmed that the antibody aggregates were removed under this condition.

### [Example B9]

The same operation as in Example B1 was performed except that the shape of the membrane was changed. Polyketone hollow fiber membrane 1 (inside diameter: 282 mm, outside diameter: 563 mm) shown in Table 2 was prepared by the method described in the paragraphs [0074] to [0077] of Patent Literature 4, and a module was prepared with an effective membrane volume of 0.15 mL. 24 mL of the antibody solution (antibody concentration: 5.05 mg/mL) was passed therethrough at a flow rate of 2.5 mL/min. The aggregate removal performance was evaluated. The results are shown in Figure 12. In Figure 12, particularly, the proportions of the aggregate (1) and the aggregate (2) compared with the monomer were decreased in fractions having M/V of 600 mg/mL or less. It was therefore confirmed that the antibody aggregates were removed under this condition.

### [Example B10]

The same operation as in Example B1 was performed except that only the method for contacting the antibody with the polyketone and the method for evaluating aggregate removal performance were changed. A polyketone powder (weight: 0.036 g, surface area: 0.10 m²) obtained by freezing and crushing the polyketone hollow fiber membrane 1 was immersed in 5 mL of the antibody solution (antibody concentration: 0.90 mg/mL), and the solution was shaken. Five hours later, the solution was collected and centrifuged for powder removal, followed by the measurement of the amounts of aggregates and an antibody concentration by the methods described in the sections (8) and (9) of Example B1. The weights of a monomer and aggregates in the collected solution were measured, and C/C0 was determined. The results are shown in Figure 13. The proportions of the aggregate (1) and the aggregate (2) compared with the monomer were decreased. It was therefore confirmed that the antibody aggregates were removed under this condition.

### (Comparative Example B1)

The same operation as in Example B1 was performed except that only the membrane used was changed. 13 mL of the antibody solution (antibody concentration: 4.87 mg/mL) was passed through a nylon membrane (Whatman(R): 7402-002, manufactured by GE Healthcare Japan Corp., pore size: 0.2 mm, diameter: 2.5 cm, membrane thickness: 160 mm, two sheets layered, effective membrane volume: 0.12 mL, surface area: 0.52 m²) at a flow rate of 0.65 mL/min. The aggregate removal performance was evaluated by the same method as in the section (9) of Example B1. The results are shown in Figure 14. In Figure 14, the concentration of the aggregate (2) compared with the monomer exceeded the initial one in fractions having M/V around 400 mg/mL. It was therefore suggested that the purification process was difficult to control.

The conditions used in Examples B1 to B9 and Comparative Example B1 are shown in Table 2.

**[Table 2]**

| | Exampl e B1 | Exampl e B2 | Exampl e B3 | Exampl e B4 | Exampl e B5 | Exampl e B6 | Exampl e B7 | Exampl e B8 | Exampl e B9 | Compara tive Example B1 |
|---|---|---|---|---|---|---|---|---|---|---|
| Pore size (um) | 0.2 | 0.2 | 0.1 | 0.4 | 0.2 | 0.1 | 0.1 | 0.1 | - | 0.2 |
| Antibody concentration (mg/mL) | 5.1 | 5.06 | 5.12 | 5.08 | 5.21 | 4.52 | 5.07 | 14.57 | 5.05 | 4.87 |
| Buffer solution | tris | Acetat e | tris | tris | tris | tris | tris | tris | tris | tris |
| Electrical conductivity (mS/cm) | 5 | 15 | 5 | 5 | 35 | 5 | 5 | 5 | 5 | 5 |
| pH | 7.0 | 5.5 | 7.0 | 7.0 | 7.0 | 7.0 | 7.0 | 7.0 | 7.0 | 7.0 |
| Flow rate (mL/min) | 0.45 | 0.45 | 0.50 | 0.60 | 0.45 | 0.5 | 3.0 | 0.50 | 2.5 | 0.65 |
| Type of antibody | CRL124 45 | CRL124 45 | CRL124 45 | CRL124 45 | CRL124 45 | AE6F4 | CRL124 45 | CRL124 45 | CRL124 45 | CRL1244 5 |
| Membrane thickness (um) | 100 | 100 | 112 | 85 | 100 | 101 | 112 | 112 | 141 | 160 |
| Effective membrane volume (mL) | 0.08 | 0.08 | 0.09 | 0.10 | 0.08 | 0.08 | 0.09 | 0.09 | 0.15 | 0.12 |
| Surface area (m2) | 0.65 | 0.65 | 1.37 | 0.40 | 0.65 | 0.94 | 1.37 | 1.37 | 3.96 | 0.52 |
| Raw material | Polyke tone | Polyke tone | Polyke tone | Polyke tone | Polyke tone | Polyke tone | Polyke tone | Polyke tone | Polyke tone | Nylon |
| Shape | Flat membra ne | Flat membra ne | Flat membra ne | Flat membra ne | Flat membra ne | Flat membra ne | Flat membra ne | Flat membra ne | Hollow fiber | Flat membran e |

## Claims

1. A method for purifying a protein-containing solution comprising a protein of interest, the method comprising the steps of:
(a1) contacting the protein-containing solution with a porous medium comprising polyketone; and
(a2) passing the protein-containing solution contacted with the porous medium comprising polyketone through a virus removal membrane.

2. A method for purifying a protein-containing solution comprising a protein of interest, the method comprising the steps of:
(b1) contacting the protein-containing solution with a porous medium having a specific surface area of 4.0 m²/mL or larger and a porosity of 50% or more; and
(b2) passing the protein-containing solution contacted with the porous medium through a virus removal membrane.

3. The method according to claim 1 or 2, wherein the porous medium is in a membrane form.

4. The method according to any one of claims 1 to 3, wherein the porous medium and the virus removal membrane are connected directly.

5. The method according to claim 4, wherein a pressure applied to the porous body used in the step (a1) or (b1) is 1/40 or more and 1/2 or less of a pressure applied to the virus removal membrane used in the step (a2) or (b2).

6. The method according to any one of claims 1 to 5, wherein filtration is performed by the application of a constant pressure.

7. The method according to any one of claims 1 to 6, wherein a virus removal rate from the protein-containing solution is at least 99.9%.

8. The method according to any one of claims 1 to 7, wherein the protein of interest is a polyclonal antibody or a monoclonal antibody.

9. The method according to any one of claims 1 to 8, wherein a protein concentration of the protein-containing solution is 100 mg/mL or lower.

10. The method according to any one of claims 1 to 9, wherein the virus to be removed with the virus removal membrane is parvovirus.

11. The method according to any one of claims 1 and 3 to 10, wherein a specific surface area (polyketone surface area (m²) / polyketone volume (mL)) of the polyketone is 12 m²/mL or larger.

12. A method for producing a protein-containing solution substantially free from a virus by a method according to any one of claims 1 to 11.

13. A prefilter for a virus removal membrane, comprising a porous medium comprising polyketone.

14. A method for recovering a purified protein from a protein-containing solution, the method comprising the steps of:
(a1) contacting the protein-containing solution with a medium comprising polyketone; and
(a2) recovering the purified protein from the protein-containing solution contacted with the medium comprising polyketone.

15. A method for selectively removing a protein aggregate contained in a protein-containing solution from the protein-containing solution, the method comprising the step of:
(b1) contacting the protein-containing solution with a medium comprising polyketone.

16. The method according to claim 14 or 15, wherein the protein is an antibody.

17. The method according to claim 16, wherein the antibody is an antibody derived from a plasma product or a cell culture solution.

18. The method according to claim 16, wherein the antibody is a monoclonal antibody.

19. The method according to any one of claims 15 to 18, wherein the protein aggregate is a dimer, a trimer, and/or a multimer of the protein.

20. The method according to any one of claims 14 to 19, wherein the step (a1) or (b1) is the step of passing the protein-containing solution through the medium comprising polyketone.

21. The method according to any one of claims 14 to 20, wherein pH of the protein-containing solution is 4 or higher and 10 or lower.

22. The method according to any one of claims 14 to 21, wherein an electrical conductivity of the protein-containing solution is 0 mS/cm or more and 100 mS/cm or less.

23. The method according to any one of claims 14 to 22, wherein a flow rate at which the protein-containing solution is passed through the medium comprising polyketone is 0.1 mL/min or more per mL of a membrane volume of the medium comprising polyketone.

24. The method according to any one of claims 14 to 23, wherein a surface area of the medium comprising polyketone is at least 0.0001 m² or larger per 1.0 g of the protein to be purified.

25. The method according to any one of claims 14 to 24, wherein the medium comprising polyketone is a porous molded body.

26. The method according to claim 25, wherein the porous molded body is a microporous membrane, a non-woven fabric, a woven fabric, a monolith, a gel, or a particle bed.

27. The method according to claim 25 or 26, wherein the porous molded body has a three-dimensionally uniform membrane pore size.

28. The method according to any one of claims 25 to 27, wherein a pore size of the porous molded body is 50 nm to 1000 nm.

29. A medium for selectively removing a protein aggregate from a protein-containing solution, the medium comprising polyketone.

30. The medium according to claim 29, wherein the protein is an antibody.

31. The medium according to claim 30, wherein the antibody is an antibody derived from a plasma product or a cell culture solution.

32. The medium according to claim 30, wherein the antibody is a monoclonal antibody.

33. The medium according to any one of claims 29 to 32, wherein the protein aggregate is a dimer, a trimer, and/or a multimer of the protein.

34. The medium according to any one of claims 29 to 33, wherein the medium comprising polyketone is a porous molded body.

35. The medium according to claim 34, wherein the porous molded body is a microporous membrane, a non-woven fabric, a woven fabric, a monolith, a gel, or a particle bed.

36. The medium according to claim 34 or 35, wherein the porous molded body has a three-dimensionally uniform membrane pore size.

37. The medium according to any one of claims 34 to 36, wherein a pore size of the porous molded body is 50 nm or larger and 1000 nm or smaller.

38. Use of polyketone for selectively removing a protein aggregate from a protein-containing solution.
